# EUROPEAN PATENT APPLICATION

(11) **EP 4 074 815 A1**
(43) Date of publication of application: **19.10.2022**
(21) Application number: 21305485.1
(22) Date of filing: 13.04.2021
(51) Int. Cl.: C12M 1/16, C12M 1/24, C12M 1/00, C12M 1/33

(54) **METHOD AND SYSTEM FOR SOLID-STATE FERMENTATION OF PLANT MATERIAL TO PRODUCE A FERMENTATION PRODUCT**

(71) Applicant: Green Spot Technologies, 31520 Ramonville-Saint-Agne (FR)
(72) Inventor: DE SARRAU, Benoit, 31520 Ramonville Saint-Agne (FR); GRANUCCI, Ninna, 31520 Ramonville Saint-Agne (FR); MUNIVE DUCTO Eramis, 31520 Ramonville Saint-Agne (FR); SICRE, Pierre, 31520 Ramonville Saint-Agne (FR)
(74) Representative: A.P.I. Conseil

(57) **Abstract**

The invention relates to a system and a method of solid-state fermentation of plant material to produce a fermentation product, the method comprising the steps of:
- providing (100) a substrate comprising plant material;
- extruding (300) the substrate having a moisture content higher than 40%;
- automatic vessels (42) filling (400) with the extruded substrate said vessels have an inner volume of 100 L or less;
- automatic inoculation (500) of the extruded substrate with a microbial inoculant adapted to cause a solid-state fermentation of the extruded substrate, the vessels (42) being in a closed state;
- storing (600) the inoculated vessels (52) during a time sufficient for a fermentation of the extruded substrate;
- harvesting (700) the content of the inoculated vessels (52), comprising the fermented substrate and microbial biomass; and
- processing (800) the harvested content to obtain a fermentation product.

## Description

### Field of the invention

The present invention relates to the field of solid-state fermentation. In particular, the invention relates to a method for solid-state fermentation of plant material.

The invention also provides a new system for solid-state fermentation of plant material.

### Description of Related Art

Solid-state fermentation (SSF) has been used for ages. Indeed, it offers several operational and economic advantages for the production of extracellular microbial products that will be purified from the fermented medium. Generally, Solid-State Fermentation may be used for the production of important biomolecules and products for many industries, including food, pharmaceutical, textile, biochemical and bioenergy. In particular, Solid-State Fermentation has many applications, such as the production of enzymes, chemicals, biogas, bioethanol and pharmaceuticals. Moreover, for some industrial applications like production of fungal spores as biocontrol agents, or extracellular feed enzymes like phytase, SSF continued to be the sole solution for the production process. Recently, in the food industry, Solid-State Fermentation has been proposed for the production of fermented flours having high nutritional interest (WO2018101844).

During Solid-State Fermentation, the growth of selected microorganism(s) occurs on solid materials. Hence, whereas submerged or liquid fermentation have notable advantages regarding instrumentation and control (monitoring of pH, dissolved oxygen, temperature, concentration of water-soluble molecules), separation of biomass after the fermentation, mixing, aeration and scaling up, these methods cannot be applied directly to Solid-State Fermentation due to the differences in the physical structures of the systems used for those respective types of fermentation.

Several studies in SSF have been devoted to the enhancement of the growth conditions in particular through the optimization of the SSF bioreactor design and engineering principles. Indeed, when efficient Solid-State Fermentation is being sought, several aspects need to be addressed such as the heat generation, oxygen availability and heterogeneity of the system. Moreover, high-solid loading in Solid-State Fermentation processes results in a series of problems such as high viscosity, high stirring energy consumption, and low conversion, which increase with the increased scale. For example, in fermentation industry, solid-state fermentation performed in culture bottles or small solid-state fermentation tanks have been considered as disadvantageous (WO2007045125). Indeed, although the storage amount of culture bottle in a certain space is increased, the shelf has no stirring, vibration, ventilation, growth monitoring and other equipment. Therefore, the whole operation process is much time-consuming, and the unwanted microbe contamination rate is high, and a large number of fermentation applications with high yield and high efficiency are difficult to do.

Hence, several bioreactor designs have been developed in an attempt to combat these problems. For example, perforated tray bioreactors have been developed. They are simple systems in which the substrate is laid out on trays, which are perforated to facilitate air convection. Use of trays for SSF addresses the heat build-up problematic by increasing the surface to volume ratio of the cultured product. However, this requires a very large storage surface, implying very large facilities, where controlling the high level of hygiene and/or axenic cultures is very difficult. Use of trays is thus not convenient for large-scale SSF. Furthermore, US6620614 discloses a solid-state fermenter, based on this technology. However, problems may also occur with oxygen transfer, which depends on tray characteristics and the height of the substrate layer. A uniform concentration of oxygen during the fermentation is difficult to obtain, because the formation of mycelium changes the porosity and thus the effective diffusivity of the states. The release of CO₂ and heat also limits the transport of O₂ and the creation of O₂ gradients is inevitable. This results in an imperfect bioreactor or complex systems to limit the drawbacks of the bioreactor.

Another concept of bioreactors for Solid-State Fermentation was also developed, which is based on continuous or intermittent stirring of the solid cultured substrate. The bioreactors may be a rotating drum or horizontal paddle mixer with or without a water jacket. Such bioreactors are expected to increase the homogeneity of the solid medium and to provide good oxygen transfer to the microorganism. They are however complex, and they may pose technical problems for large-scale use concerning inoculation, sterilization of the substrate, harvest and cleaning in/of the bioreactor. Morevover, when it come to fungi, some fungi species are sensitive to shear and breaking of mycelia makes it impossible to use the systems.

Such previously described attempts can have some advantages when the production of a particular enzyme or by-product that will be purified is sought. However, in the agri-food industry, when the production of fermentation products at low costs is expected, such systems or process are not relevant. Moreover, an impaired growth during the Solid-State Fermentation can induce the production of fermentation by-products that will be damaging to the quality of a full batch of fermentation products.

Hence there is a need for systems or process enabling SSF capable of producing, from plant material, a fermentation product, for example a fermented edible product, while controlling cost. There is also a need for new methods and systems to produce large quantities of fermentation products which are fermented from a greater range of substrates without having to use large and complex bioreactors arranged to control the fermentation through forced aeration, mixing, temperature gradients controls, water addition to compensate heat and dehydration during fermentation or on-line measurements.

### Summary of the Invention

The following sets forth a simplified summary of selected aspects, embodiments and examples of the present invention for the purpose of providing a basic understanding of the invention. However, the summary does not constitute an extensive overview of all the aspects, embodiments and examples of the invention. The sole purpose of the summary is to present selected aspects, embodiments and examples of the invention in a concise form as an introduction to the more detailed description of the aspects, embodiments and examples of the invention that follow the summary.

The invention aims to overcome the disadvantages of the prior art. In particular, the invention proposes a method or system of solid-state fermentation of plant material, said method or system allowing the preparation a fermentation product, for example a fermented edible product. In particular, an extruded substrate to be fermented is filed in vessels, said vessels are closed and can be stored in a storage area without aseptic atmosphere. By such a combination of features, it is possible to conduct an efficient fermentation without complex systems controlling the fermentation for example by forced aeration, mixing, water addition to compensate heat and dehydration during fermentation or temperature gradients controls. Thus, this fermentation product can be produced in large quantities, without the drawbacks of the systems known in the state of the art.

Hence, according to an aspect of the present invention, it is provided a method of solid-state fermentation of plant material to produce a fermentation product, said fermentation product comprising fermented plant material, the method comprising the steps of:
- providing a substrate to be fermented, said substrate comprising plant material;
- extruding the substrate to be fermented, said extruded substrate to be fermented having a moisture content higher than 40%;
- automatic vessels filling with the extruded substrate to be fermented, said vessels having an inner volume of 100 L or less;
- automated inoculation of the extruded substrate with a microbial inoculant adapted to cause a solid-state fermentation of the extruded substrate, the inoculated vessels being in a closed state after the inoculating step;
- storing the inoculated vessels in a closed state, preferably in controlled climate conditions, during a time sufficient for the fermentation of the extruded substrate;
- harvesting of the content of the inoculated vessels, said content comprising the fermented substrate and microbial biomass; and
- processing the harvested content to obtain a fermentation product, preferably a fermented edible product, from all or part of the content of the inoculated vessels.

The method and the system developed in the invention makes it possible to produce a fermentation product comprising fermented plant material. This fermentation product can be produced in large quantities, without the drawbacks of the systems known in the state of the art. The traditional approach followed in the state of the art to scale-up axenic fermentation process consists in the development of large bioreactors with forced aeration, mixing, water addition to compensate heat and dehydration during fermentationor temperature gradients controls. Conversely, the present invention is based on the use of an increased number of simple and small reactors called vessels in which is performing a solid-state fermentation.

This result, based on a counter-intuitive approach in the field of bioprocessing, is obtained by providing several small and closed vessels filed with an extruded substrate with controlled moisture content, said small and preferably closed vessels being preferably manipulated by using an automated system instead of increasing the size of the reactors. Indeed, the less favorable growth conditions resulting from the use of small vessels that can be handled and moved to an automated fermentation system compared to large conventional bioreactors are counterbalanced by the extrusion step and the moisture content higher than 40%. Substrates extrusion and moisture content are important to control vessels filling and overall fermentation. It allows a better dispersion of the substrates into the vessels allowing the microbial inoculant to grow in a faster and more homogeneous way. Moreover it decreases latency time between inoculation of the microbial inoculate and growth start, leading to a faster and better colonization of the substate.

The invention results in a process, which has many advantages over the known processes, in terms of fermentation speed and reduction of contamination. In addition, it can also permit versatility in substrates, as some substrates could not be used efficiently without extrusion.

The invention is particularly adapted to the production of a fermentation product, preferably a fermented edible product, such as a fermented flour for the food industry.

**According to other optional features of the method of solid-state fermentation of plant material, it can optionally include one or more of the following characteristics alone or in combination:**
- the moisture content of the extruded substrate, preferably before the automatic vessels filling, is comprised between 45% and 85%. Such a moisture content as illustrated in example allow an improve of the fermentation combined with extrusion and vessels of 100L or less.
- the moisture content of the extruded substrate, preferably before the automatic vessels filling, is at least 50%. Such a moisture content as illustrated in example allow an improve of the fermentation combined with extrusion and vessels of 100 L or less.
- the extrusion of the substrate to be fermented is conducted through opening having a cross section with an area value smaller than 2 cm². Such area value of cross section allows good fermentation rate.
- the substrate to be fermented comprises peels, skins, pulp, seeds, pomace, cake, marc, straw, bran, cobs, shells, and/or kernel of fruit, vegetable, cereal and/or pulse. Preferably, the substrate to be fermented comprises peels, skins, pulp, seeds, pomace, cake, and/or marc of fruit, vegetable, cereal and/or pulse. More preferably, the substrate to be fermented comprises peels, skins, pulp, seeds, pomace, cake, and/or marc of fruit or vegetable. When the extrudate substrate comprises fruit and/or vegetable by-products the fermentation, in a method according to the invention, is enhanced.
- the substrate to be fermented comprises plant material from plants of different families. Combination of plant material can beneficial for fermentation.
- the automatic filling step is done at a rate of at least fifty vessels per hour. Such a rate is facilitated by the automation of many steps, the size of the vessels and the use of an extrusion step.
- a step of killing microorganisms before the automatic inoculation step occurs, said step of killing microorganisms being applied to empty vessels, filed vessels, plant material and/or the substrate to be fermented before the filing of the vessels. Killing microorganisms before inoculation can enhance the fermentation.
- the fermentation product is preferably selected from: liquid preparations such as beverage, gel/paste preparations, or powders preparations such as flours. More preferably, the fermentation product comprises: spores, enzymes, pigments, flavours, fragances, microbial biomass, and/or antimicrobials. The method of the invention allows the production of a wide variety of products.
- a step of closing the vessels with a barrier system after the step of filling and/or after the step of inoculation. For example, the inoculation step comprises seeding the microbial inoculant on the substrate when the vessel is closed (for example through a wall of the vessels, such as polymer septum), or when the vessel is opened. Such a step limit contamination of the extruded substrate.
- The microbial inoculant adapted to cause a solid-state fermentation of the substrate comprises at least one microorganism belonging to the taxonomic class *Bacilli.* This method of fermentation for large-sclae fermentation was never proposed for these microorganisms.
- The microbial inoculant adapted to cause a solid-state fermentation of the substrate comprises at least one microorganism belonging to the genus *Brettanomyces* or *Aureobasidium.* This method of fermentation for large-sclae fermentation was never proposed for these microorganisms.
- The microbial inoculant adapted to cause a solid-state fermentation of the substrate comprises at least one Dikarya fungus. This method of fermentation for large-scale fermentation was never proposed for these microorganisms. Moreover, the microbial inoculant can comprise a combination of microorganisms belonging to same or different the taxonomic classes.
- The inoculated vessels in the closed state are stored in a non-aseptic environment. Hence, the conditions for controlling the sterility of this environment are reduced compared to other methods where the vessels could remain open.
- The closed state allows gaseous exchanges between the inside of the inoculated vessels and the outside, through a barrier system. This allow a better fermentation.

According to another aspect of the invention, it is provided a system for solid-state fermentation of plant material to produce a fermentation product, said fermentation product comprising fermented plant material, said system is preferably capable of performing a method according to the invention, said system comprising:
- a supply device arranged to receive a substrate to be fermented, said substrate to be fermented comprising plant material;
- an extruder arranged to extrude the substrate to be fermented at a moisture content higher than 40%;
- an automated filling device arranged to fill vessels with the extruded substrate, said vessels having an inner volume of 100 L or less;
- an automatic inoculating device of the extruded substrate with a microbial inoculant adapted to cause a solid-state fermentation of the extruded substrate; and
- an incubation chamber arranged to allow a solid-state fermentation of the extruded substrate by the microbial inoculant in the inoculated vessels, the vessels being in a closed state.

Preferably the system also comprises:
- an automated harvesting device arranged to harvest the content of the inoculated vessels, said content comprising the fermented substrate and microbial biomass; and
- an automated processing device configured to process the harvested content to obtain a fermentation product from all or part of the content of the inoculated vessels.

**According to other optional features of the system of solid-state fermentation of plant material, it can optionally include one or more of the following characteristics alone or in combination:**
- a device adapted to kill microorganisms. Such a device, configured to kill microorganisms, for example in the filled vessel, before inoculation can enhance the fermentation.
- an automatic conveying system such as a conveyor belt. For example, automated guided vehicles may be used.
- The supply device is arranged to receive a substrate to be fermented, preferably comprising mainly plant material. Preferably, the supply device is configured to provide a substrate to be fermented at least 100 kg per hour.
- A vessel wall allowing seeding the microbial inoculant on the substrate through it.
- A barrier system allowing gaseous exchanges between the inside of the inoculated vessels and the outside.

### Brief description of the drawings

The foregoing and other objects, features and advantages of the present invention will become more apparent from the following detailed description when taken in conjunction with the accompanying drawings in which:
FIG. 1 is a schematic view showing an embodiment of a method of solid-state fermentation of plant material to produce a fermented edible product according to the invention. Facultative steps are represented in dotted frames and may be implemented in parallel with other steps, and/or at the end.
FIG. 2 is a schematic view showing a substrate preparation according to the invention.
FIG. 3 is a schematic view showing a substrate inoculation according to the invention.
FIG. 4 is a schematic view showing fermented substrate post treatments according to the invention.
FIG. 5a and FIG. 5b represent example embodiments of vessels that may be used in a method or in a system according to the invention.
FIG. 6 is a schematic view showing an embodiment of a system of solid-state fermentation of plant material to produce a fermented edible product according to the invention. Facultative devices are represented in dotted frames.
FIG. 7 is a schematic view showing an automated system according to an embodiment of the invention.

The figures do not necessarily respect the scales and are for illustration purposes.

Aspects of the present invention shall be described with reference to flow diagrams and/or block diagrams of methods, apparatus (systems) and computer program products according to embodiments of the invention.

In the figures, flow charts and block diagrams illustrate the architecture, functionality and operation of possible implementations of systems and methods according to various embodiments of the present invention. In this respect, each block in the flowcharts or block diagrams may represent a system, device, module or code, which comprises one or more executable instructions for implementing the one or more specified logical functions. In some implementations, the functions associated with the blocks may appear in a different order than shown in the figures. For example, two blocks shown in succession may, in fact, be executed substantially simultaneously, or the blocks may sometimes be executed in reverse order, depending on the functionality involved. Each block in the block diagrams and/or flowchart, and combinations of blocks in the block diagrams and/or flowchart, may be implemented by special hardware systems that perform the specified functions or acts or perform combinations of special hardware and computer instructions.

### Detailed description

A description of example embodiments of the invention follows.

In the following description, **"solid-state fermentation"** means a technique for growing microorganisms, such as fungi, yeast and bacteria, on solid substrates, and preferably in connection with food production. A solid-state fermentation may also refer to the process of fermenting microorganisms on a solid medium that provides anchorage points for the microorganisms in the absence of any freely flowing substance.

As used herein, **"plant material"** can be derivable from any plant and plant part, including tubers, roots, stems, leaves, fruits and seeds. Materials that can be usefully processed using the methods described herein include cellulosic and lignocellulosic materials, e.g., arable products, crops, grasses, plants and/or feed grains, for example including, but not limited to, plant material (e.g., forage such as alfalfa meals, hay, Bermuda coastal grass hay, sweet grass, corn plants, and soybean hay), grains (e.g., barley, corn (including organic and genetically modified corn), oats, rice, sorghum, and wheat), plant protein products (e.g., canola meals, cottonseed cakes and meals, safflower meal, and soybean (including organic and genetically modified soybean) feed and meal), processed grain by-products (e.g., distillers products, brewers dried grains, corn gluten, sorghum germ cake and meals, peanut skins, and wheat bran), fruit and fruit by-products (e.g., dried citrus pulp, apple pomace, and pectin pulp), molasses (e.g., beet, citrus, starch, and cane molasses), almond hulls, ground shells, buckwheat hulls, legumes and legume by-products, and other crop by-products. Other raw materials include, but are not limited to, alfalfa, barley, birdsfoot trefoil, brassicas (e.g., chau moellier, kale, rapeseed (canola), rutabaga (swede), and turnip), clover (e.g., alsike clover, red clover, subterranean clover, and white clover), grass (e.g., false oat grass, fescue, Bermuda grass, brome, heath grass, meadow grass, orchard grass, ryegrass, and Timothy grass), invasive plants, maize (corn), millet, oats, sorghum, and soybeans. Preferably, "plant material" refers to any plant or plant part of plant selected from: orange, grape, potatoes, sugarbeet, apple

The expression **"fermentation product",** within the meaning of the invention, can correspond to products generated during a fermentation step according to the invention. A fermentation product can correspond to fermented substrate, spore, biomass such as mycelium, molecules or a mix thereof.

By **"edible product",** it should be understood any product that is orally taken by animals and/or human beings, in a solid or liquid form, such as flour and products derived from flour, breadmaking products, biscuits, cakes, meat substitutes, dairy products (milk, yogurt, and butter), dairy substitutes, beverages, cooking aid (stock cubes and the like, aroma, emulsifying agent...)...

In particular, the expression **"fermented edible product",** within the meaning of the invention, can correspond to any edible product which includes a fermentation product.

By **"substrate",** it should be understood any composition comprising organic matter or a mix between organic matter and inorganic matter allowing initiating or facilitate a fermentation process, for example it can be a source of glucose, a source of carbon, a source of protein, or more generally a source of nutrients that may comprise carbon, nitrogen, minerals... In addition, a substrate may comprise any plant material.

In particular, the expression **"substrate to be fermented",** within the meaning of the invention, can correspond to any substrate suitable for the development of an organism responsible for the fermentation process.

In the following description, **"vessels"** may refer to any container or recipient adapted to receive a substrate to be fermented by a microorganism in solid-state fermentation. Preferably, a vessel according to the invention will be moveable by automated transportation device such as conveyor belts. Moreover, a vessel according to the invention is arranged such that it can be stored in a closed state allowing gaseous exchanges but preventing microbial contamination of the extrudate substrate.

By **"closed state",** it should be understood any system allowing solid-state fermentation to be carried out in such a way that fermenting microorganisms and the fermentation products it produces are kept as an axenic culture during fermentation as a fermentation containment.

By **"barrier system",** it should be undersood technical means to close the vessels while allowing gazeous exchanges between inside and outside of the vessels. Those means could be a membrane, a cap, a lid, a filter, a porous material, such as a porous membrane or any conventional gaz permeous solution, preferably forming a microbial filter. In particular, as described hereafter, a barrier system can be used to prevent the entry of particles that could cause deleterious effect to the fermentation.

The term **"automated"** according to the invention can be understood as any automatized system for performing operations, happening without the participation of the will, acting or doing spontaneously. Automatic transport refers to a transport by an automatic conveying system, such as a conveyor belt, a lift system or an automatic guided vehicle.

**"By-product"** generally relates to a product that is produced as a result of making another product. For example, by-products can be produced during the extraction of juice from fruits or vegetables. Pomace is a typical by-product of fruit juice and vegetable (tomato, carrot) production; sugar beet pomace is a by-product of beet sugar production. Peels and seeds are also common by-products of the food industry. Hence by "by-product » it can be understood peels, skins, pulp, seed, pomace, cake, marc, straw, bran, cobs, shells, and kernel of: fruit, vegetable, cereal or pulse.

By **"automated filling device",** it should be understood any robotic handling device exhibiting filling uniformity, and which can be programmed, such as filling speed and filling rate.

By **"automated inoculation station",** it should be understood any robotic handling device exhibiting act or process or an instance of inoculating, with a predetermined microbial inoculant (e.g. solid form, liquid form or other form), and which can be programmed as the quantity of microbial inoculant to be introduced in the vessels.

By **"automated harvesting device",** it should be understood any robotic handling device capable of removing, extracting, picking, or separating the content of the vessels after the solid-state fermentation or depositing harvested content for conveying to post-harvesting. Automated harvesting device may also take in charge spores, or liquid from substrate.

The term **"microbial inoculant"** according to the invention can be understood as a composition comprising microorganisms to be added to the extruded substrate. The composition can be in solid form, liquid form, or other form (e.g. suspension) or a mix thereof.

In particular, the expression **"capable of solid-state fermentation"** within the meaning of the invention, can correspond to capacity (i.e. of a microorganism) to achieve a fermentation process in which a solid material is used as a physical support as well as a nutrient source for the growth of microorganisms.

In particular, the expression **"Dikarya fungus",** within the meaning of the invention, can correspond to any species of fungus belonging to a phylum selected from *Ascomycetes* or *Basidiomycetes.*

The expression **"controlled climate conditions",** within the meaning of the invention, can correspond to a type of control unit that is specially designed to maintain steady temperatures, humidity levels and CO₂ and O₂ (and eventually other gases). Temperature, humidity and CO₂ and O₂ are important metrics when it comes to optimizing the climate in a space because they work together to create optimal conditions. Controling temperature,humidity and CO₂ and O₂ also allow optimizing the development of microorganisms in general.

The expression **"aseptic conditions", "hygienic conditions"** or **"axenic conditions",** within the meaning of the invention, can refer to an environment that has been made contamination-free, which means that the environment will not allow any kind of harmful living microorganisms to reproduce, more particularly harmful and/or deleterious bacteria, viruses or fungi except the microorganism of interest (axenic conditions). In particular, contrary to sterilization where all living microorganisms are destroyed, in the meaning of the invention, the expressions **"aseptic conditions"** and **"hygienic conditions"** can include keeping microorganisms considered helpful and/or that doesn't have a deleterious effect. In the meaning of the invention, the expression **"axenic conditions"** can refer to a culture in which only a single species, variety, or strain of organism is present.

The expression **"non-aseptic conditions",** within the meaning of the invention, can refer to an environment where the presence of any kind of harmful living microorganisms, more particularly harmful and/or deleterious bacteria, is not controlled nor regulated.

The term **"rigid"** can refer to a component that does not flex or bend without irreversible changes when subject to typical forces experienced by a vessel during a fermentation process. A rigid component has a tensile modulus equal to at least 10 Megapascals (MPa), preferably to at least 20 MPa. The term **"flexible"** refers to a component that easily flexes or bends when subject to typical forces experienced by a a vessel during a fermentation process. A flexible component has a lower tensile modulus than a rigid component, e.g. lower than 10 Megapascals. Rigidity may be measured according to ISO 527-1:2019, ISO 527-3:2018 or ISO 178:2019, which are standard methods for determining tensile properties and flexural properties. Specifically, rigidity may be measured at a preferred atmospheric temperature (ISO 527-1 , clause 8), at a preferred test speed (ISO 527- 1 , clause 5 and ISO 527-3, clause 5), and with a preferred test specimen (ISO 527-3, clause 6), as described in these ISO standard methods.

When introducing elements of various embodiments of the present disclosure, the articles "a," "an," and "the" are intended to mean that there are one or more of the elements. The terms "comprising," "including," and "having" are intended to be inclusive and mean that there may be additional elements other than the listed elements.

Additionally, it should be understood that references to "one embodiment" or "an embodiment" of the present disclosure are not intended to be interpreted as excluding the existence of additional embodiments that also incorporate the recited features.

As mentioned, traditionally solid-state fermentation, used mainly for enzyme production, is carried out in trays or packed-bed bioreactors and recently in rotating drums, fluidized beds or gas-solid spouted bed bioreactors. Hence, in the last years, for more steady production of enzymes, it has been developed more complex bioreactors capable of controlling fermentation by forced aeration, building temperature gradients capable of removing metabolic heat and of on-line measurements to control the fermentation.

The applicant specialized in solid-state fermentation for the production of fermentation products and preferably of fermented edible products developed a new solution based on simple vessels that can be integrated in an automated system, filed with an extruded substrate with a controlled moisture content, and closed to be fermented. In particular, the applicant developed a solution where the extrusion of the substrate combined with simple vessels, that can be closed, allows an efficient fermentation without complex systems controlling the fermentation by forced aeration or temperature gradients. Advantageously, the applicant developed a solution which permits the use of a number of plant materials that if not extruded impose a great challenge to scale up with solid-state fermentation technology.

The method and the system illustrated in the present invention make it possible to efficiently produce a fermentation product which is fermented from plant material. This fermentation product, for example a fermented edible product, can be produced in large quantities, without the drawbacks of the systems known in the state of the art. Contrary to the approach followed in the state of the art to scale-up fermentation process and consisting of the development of large and complex bioreactors, the present invention is based on a combination of features to perform an efficient and not expensive solid-state fermentation.

Indeed, it has been discovered that vessels filed with an extruded substrate comprising plant material and with a controlled moisture content can support an efficient solid-state fermentation compared to vessels filed with a non-extruded substrate or not controlled moisture content. Moreover, as illustrated by examples, it has been found that the extrusion can enhance, depending on the substrate, the fermentation by more than 90% and permit the use of a number of plant material that if not extruded will impose a great challenge to scale up solid-state fermentation.

Hence, the solution according to the invention helps fermenting a great range of plant materials in solid-state fermentation and obtaining better industrial results without using complex and large bioreactors. Indeed, the solution according to the invention is easily transposable on an industrial scale, not expensive, simple to use, and efficient in particular when scaled-up.

According to a **first aspect,** the invention relates to a method 1 **of solid-state fermentation of plant material to produce a fermentation product, for example a fermented edible product.**

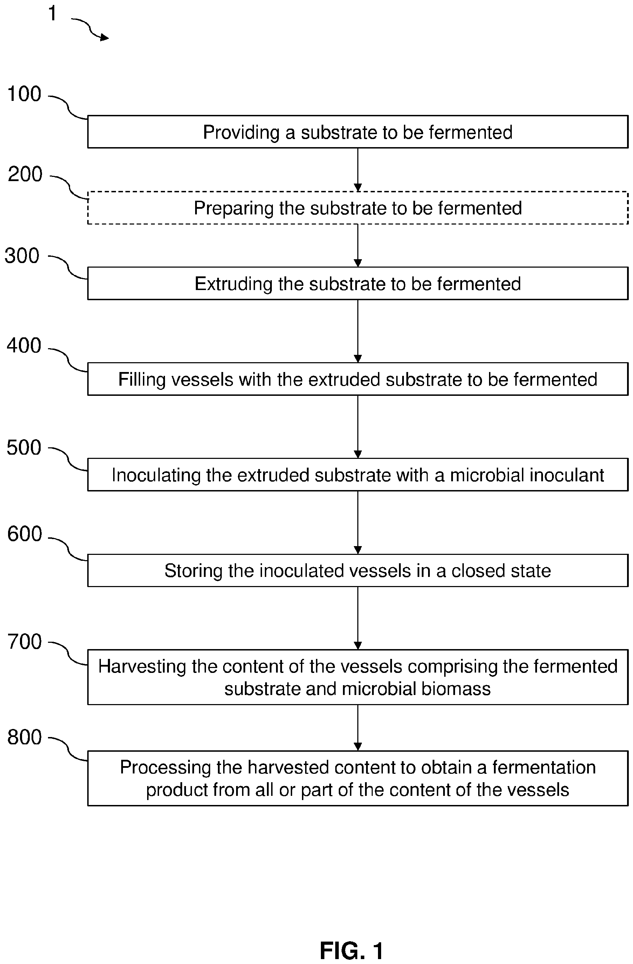

As shown in figure 1, said method comprises the steps of: providing 100 a substrate to be fermented, extruding 300 the substrate to be fermented, vessels filling 400 with the extruded substrate to be fermented, inoculating 500 the extruded substrate with a microbial inoculant; storing 600 the inoculated vessels in a closed state; harvesting 700 the content of the inoculated vessels comprising the fermented substrate and microbial biomass (i.e. microbial biomass from the microorganism(s) inoculated with the microbial inoculant); and processing 800 the harvested content to obtain a fermentation product.

As shown in figures 1, 2, 3, and 4, said method can also comprise the steps of: substrate pre-processing 200, such as sorting 210 of the plant material, drying 220 the plant material, grinding 230 the plant material; Opening 510 the vessels, seeding 520 the microbial inoculant in the vessels, closing 530 the vessels; Drying 810 the fermentation product, grinding or milling 830 the fermentation product and conditioning 830 the fermentation product.

As it will be described, a method 1 according to the invention can be considered as a large-scale solid-state fermentation of plant material to produce a fermentation product. In particular, a method 1 of solid-state fermentation of plant material according to the invention can comprise automated steps for the manipulation of the vessels. Hence, preferably, a method 1 of solid-state fermentation of plant material according to the invention comprises at least one of the steps of filling, inoculating, and harvesting performed by automates; more preferably a method 1 of solid-state fermentation of plant material according to the invention comprises at least two of the steps of filling, inoculating, and harvesting performed by automates; even more preferably a method 1 of solid-state fermentation of plant material according to the invention comprises the steps of filling, inoculating, and harvesting performed by automates. Indeed, the use of high-volume bioreactors can be replaced in the present invention by the combined use of vessels, automated manipulation of vessels and extruded substrate. The method may be continuous or performed by batches. The method may mix continuous steps (i.e. steps performed continuously without planned interruptions) and steps performed by batches of vessels. For example, as it will be described hereafter, the sterilizing step may be performed by batches of at least 100 vessels whereas the filling step is done continuously at a rate of at least 50 vessels per hour.

A first step of the method according to the invention can be a **step of providing 100 a substrate to be fermented 12.** As it will be described hereafter said substrate to be fermented 12 comprises advantageously plant material.

The substrate to be fermented 12 can advantageously be provided through a supply device 10. The supply device 10 can for example be a supply hopper. Alternatively, it can be an automated supply device such as an endless screw. As the developed method is highly suitable for high scale fermentation of plant material, the step of providing 100 a substrate to be fermented 12 can provide at least 100 kg per hour of a substrate to be fermented 12, preferably at least 500 kg per hour of a substrate to be fermented 12, more preferably at least 1000 kg per hour of a substrate to be fermented 12 and even more preferably at least 2000 kg per hour of a substrate to be fermented 12.

The provided plant material is generally from agri-food industry. The agri-food industry can for example encompasses waste material produced during agricultural practice, food industry, feed industry, pet food industry, and/or by-products of the industries just mentioned.

For example, the plant material is a by-product of the agri-food industry. Hence, the substrate to be fermented 12 can comprise fruit(s) or/and vegetable(s). In particular, it can comprise fruit(s) or/and vegetable(s) by-products such as skins, peels, pomace, seeds, pulp, marc, straw, bran, cobs, shells, and/or kernel. Preferably, it can comprise fruit(s) or/and vegetable(s) by-products such as skins, peels, pomace, seeds, pulp, and/or marc.

Preferably, the substrate to be fermented 12 comprises mainly, in weight, fruit(s) or/and vegetable(s). Preferably, the substrate to be fermented 12 comprises at least 40% by weight of fruit(s) or/and vegetable(s), more preferably at least 50% by weight of fruit(s) or/and vegetable(s), even more preferably at least 60% in weight by fruit(s) or/and vegetable(s). The substrate to be fermented 12 can comprise 80% or more, in weight, such as more than 90% in weight, of fruit(s) or/and vegetable(s).

In particular, such fruit(s) or/and vegetable(s) can comprise by-product(s) of the agri -food industry such as skins, peels, pomace, seeds, pulp, marc, straw, bran, cobs, shells, and/or kernel of fruits and/or vegetables. Hence, in an example, the substrate to be fermented 12 can comprise at least 40% by weight of peels skins, pulp, skins, pomace, and/or seeds of fruits and/or vegetables. The substrate to be fermented 12 can comprise at least 50% by weight of fruit(s) or/and vegetable(s) by-product(s), even more preferably at least 60% in weight by fruit(s) or/and vegetable(s) by-product(s). The substrate to be fermented 12 can comprise 80% or more, in weight, such as more than 90% in weight, of fruit(s) or/and vegetable(s) by-product(s).

The substrate to be fermented 12 can also comprise cereal(s) or/and pulse(s), preferably in addition of the peels, skins, pulp, skins, pomace, and/or seeds of fruits and/or vegetables.

For example, the substrate to be fermented 12 can comprise acorn squash, açai, alfalfa sprouts, allium (leeks, chives, garlic, garlic chives), amaranth, angelica, anise, anise hyssop, apple, apricot, artichoke, arugula, asian pear, asparagus, atemoya, avocado, azuki beans (or adzuki), bachelor's button, bamboo shoots, banana, banana squash, barley, basil, bean sprouts, beans, bee balm, beets, belgian endive, bell peppers, berries, bitter melon, black beans, black mustard, blackberries, black-eyed peas, blueberries, bok choy, boniato, borage blossoms, borlotti bean, boysenberries, broad beans, broccoflower, broccoli, brussels sprouts, buckwheat (smartweed family), butternut squash, cabbage, cactus pear, calendula/marigold, canary grass, cantaloupe, carambola, caraway, carnations/dianthus, carrots, casaba, cassava, melon, cauliflower, cayenne pepper, celery, chamomile, chamomile, chayote, cherimoya, cherries, chervil, chia, chickpeas, chicory, chili pepper, chives, chrysanthemum, cilantro, citrus (orange, lemon, lime, grapefruit, pomelo, mandarin, kumquat, etc.), clover flowers, coconuts, coix lacryma-jobi var. ma-yuen, collard greens, common beans, common peas (garden peas), coriander, corms, corn, courgette, cranberries, cucumber, daikon, dandelion, dates, delicate, dill, durum wheat, dried plums, eddoe, eggplant, endive, english daisy, escarole, fava beans, feijoa, fennel, fiddleheads, figs, finger millet, flax seed (flax family), fonio, foxtail millet, frisee, fuchsia, garlic, gem squash, ginger, gladiolus, gooseberries, grapefruit, grapes, green beans, green onions, greens, guava, habanero, hemp seed (hemp family), hibiscus, hollyhock, hominy, honeydew melon, horned melon, horseradish, hubbard squash, iceberg lettuce, impatiens flowers, india mustard, jalapeño, japanese millet, jasmine, jerusalem artichoke, jicama, Job's Tears, Johnny jump-up, kale, kañiwa, kidney beans, kiwicha, kiwifruit, kodo millet, kohlrabi, konjac, kumquat, lavender, leeks, lemon grass, lemon verbena, lentils, lettuces, lilac, lima beans, limes, longan, loquat, lupins, lychee, matcha, madarins, maize, Malanga, mangel-wurzel, mangetout or snap peas, marjoram, marrow mangos, millet, mint, mulberries, mung beans, mustard, mustard greens, napa, nasturtium, navy beans, nectarines, nettles, new zealand spinach, oats, okra, onion, oregano, papayas, paprika, parsley, parsnip, passion fruit, peaches, peanuts, pearl millet, pears, peas, peppers, persimmons, pigeon peas, pineapple, pinto beans, pitseed goosefoot, plantains, plums, pomegranate, poppy seed, potatoes, prickly pear, proso millet, prunes, pumpkin, quince, quinoa, radicchio, radish, radishes, raisins, rapeseed (including canola), raspberries, red cabbage, rhubarb, rice, romaine lettuce, rose, rosemary, runner beans, rutabaga, rye, safflower, sage, sage blossoms, salsify (oyster plant), scallion, shallots, skirret, snow peas, sorghum, soybeans, spaghetti squash, spelt, spinach, sprouts, squash, strawberries, string beans, sugarcane, sugar beet, sunflower, sweet potato, sweetcorn, tabasco pepper, tangelo, tangerines, taro, tatsoi, teff, tiger nut, thyme, tomatillo, tomato, topinambur, triticale, turnip, ugli fruit, valerianella, violets, wasabi, water chestnuts, watercress, watermelon, waxed beans, wheat, white radish, wild rice, yams, yellow squash, yuca/cassava, zucchini squash.

Preferably, the substrate to be fermented 12 can comprise apple, apricot, artichoke, arugula, asian pear, bamboo, banana, banana squash, barley, beets, broccoflower, broccoli, brussels sprouts, cactus, carrots, casaba, cassava, citrus (orange, lemon, lime, grapefruit, pomelo, mandarin, kumquat, etc.), cauliflower, coconuts, common beans, common peas (garden peas), corms, corn, courgette, cucumber, cranberries, cucumber, dried plums, eddoe, eggplant, endive, garlic, gem squash, ginger, gladiolus, gooseberries, grapefruit, grapesmelon, rice, string beans, sorghum, soybeans, sugarcane, sugar beet, sunflower, sweet potato, sweetcorn, tomato, topinambur, water chestnuts, watercress, watermelon, wheat, or wild rice.

The substrate to be fermented 12 can comprise plant material from plants of different families. For example, the substrate to be fermented 12 can comprise a mix of several vegetables, a mix of several fruits or a mix of vegetables and fruits, a mix of fruits and/or vegetables and/orcereals and/or pulses and/or by-products of all these.

Advantageously, the substrate to be fermented consists mainly of skins/peels, pulp, seeds, pomace, cake, and/or marc. Pomace is usually a term to designate the combination of all the solids that are left after the juice extraction, cake for the case of olive, canola, soy, sunflower, marc in the case of grapes.

The used by-products may depend on the production area and on the season, i.e. the period of the year. The process may use by-products having a low value, or by-products difficult to exploit by the agri-food industry. For example, grape marc is widely available in the wine regions in autumn.

Solid-state fermentation of fruit and vegetable by-products is usually hindered by a very high heterogeneity of the substrates and high moisture levels of the substrates combined with high content of simple sugars (e.g. glucose, fructose, sucrose, etc) making these substrates very stick with poor porosity. These limiting factors are even more challenging at industrial scale up when large amount of substrate needs to be fermented. Therefore, scale up by increasing the number of vessels is particularly advantageous when using these types of substrates. As it will be described, the used vessels preferably have specific dimensions for an optimization of the fermentation method and are combined with the use of an extruded substrate.

The moisture content of the plant material can be from 5 % to 98 % in weight. As the method comprises a step of extruding 300 and eventually a step of drying 220 the plant material, the moisture content of the plant material 12 has no real impact on the method.

A method according to the invention can comprise **a step of preparing 200 the substrate to be fermented 12, such as the plant material.**

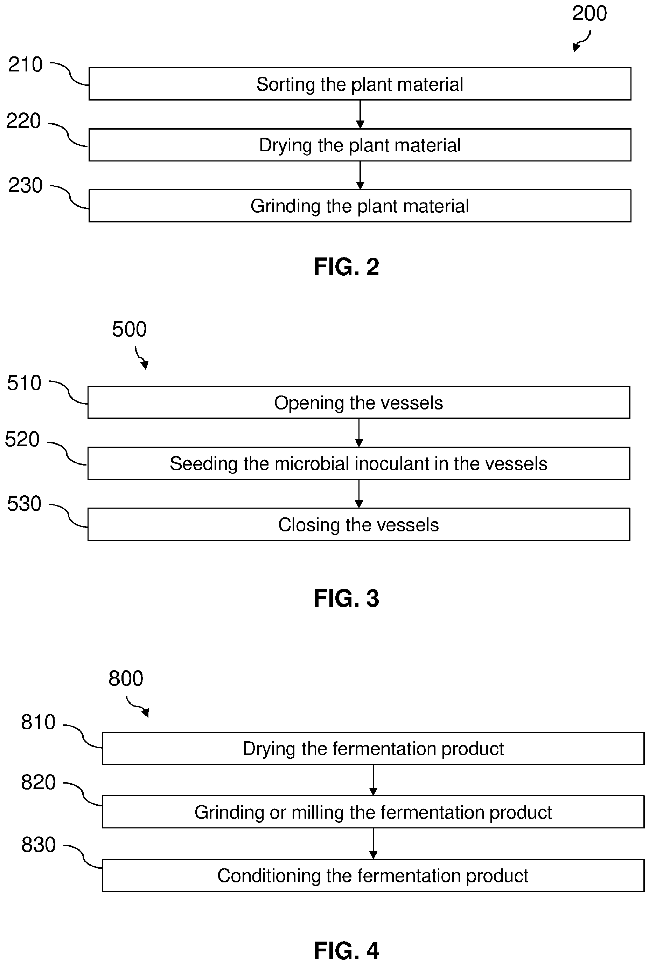

As illustrated in figure 2, a method according to the invention can comprise a step of sorting 210 of the plant material. In particular, the step of sorting 210 will allow rejecting undesirable material, such as plastics or plant material that is too large. For example, the step of sorting 210 can be configured to reject plant material having a smallest dimension bigger than 5 mm.

A method according to the invention can comprise a step of drying 220 the plant material. In particular, the step of drying 220 will allow reducing water content of plant material especially when it is above 85%, and in particular when it is greater than 95%. For example, the step of drying 220 can be configured to apply heat treatment at a temperature greater than 40°C to the plant material.

As illustrated in figure 2, a method according to the invention can comprise a step of grinding 230 the plant material. In particular, the step of grinding 230 will allow reducing the particle size plant material. For example, the step of grinding 230 can be configured to produce particles of plant material having a D50 (D50 is the size in microns that splits the distribution with half above and half below this diameter) comprised between 0.01 mm and 15 mm, preferably below 10 mm.

In particular, at this step, the substrate is prepared or provided. Preparing the substrate may comprise for example crushing, boiling, steaming, drying, hydrating, pressing, washing or mixing raw materials. The substrate to be fermented 12 corresponds to the culture material where a microorganism will develop and cause a solid-state fermentation.

A method according to the invention comprises **a step of extruding 300 the substrate to be fermented 12.**

The step of extruding 300 the substrate to be fermented 12 can be broadly described as a step of forcing the substrate to be fermented 12 through an opening. During the step of extruding 300, generally involving at least one screw and a die, high pressure can build up at the end of the screw and the die. In particular, it can be considered that this extrusion step combines various unit operations into one system: transport, particle size reduction, particle size alteration, shape alteration, moisture alteration (increase or decrease), mixing, extraction, washing, cooling and/or heating, steaming, compression and/or expansion of the material.

The use of "small" vessels 42 (100 L or less) that can preferably be handled and moved to an automated fermentation system induces growth conditions which are less favorable than the conditions which can be found in conventional bioreactors in which temperature gradients will be controlled and oxygenation reinforced. In the context of the present invention, this extrusion step makes it possible to prepare the substrate to be fermented 12 and and counterbalance these suboptimal growth conditions.

In particular, the step of extruding 300 the substrate to be fermented 12 can comprise exerting a pressure higher than 1 bar on the substrate to be fermented 12, preferably of at least 5 bars on the substrate to be fermented 12, more preferably exerting a pressure of at least 10 bars on the substrate to be fermented 12, even more preferably exerting a pressure of at least 20 bars on the substrate to be fermented 12, for example exerting a pressure of at least 50 bars on the substrate to be fermented 12.

The extrusion processing can involve a lot more than just forcing the substrate to be fermented 12 through an opening. In particular, the substrate to be fermented 12 can be subjected to relatively high levels of shears in an extruder, generating temperatures higher than 40°C, which leads to mixing, compaction, phase transition, and eventually molecular breakdown of the substrate to be fermented 12.

The step of extruding 300 the substrate to be fermented 12 can comprise a step of cooling the substrate to be fermented 12 in order to maintain a temperature of the substrate to be fermented 12 below 40°C. However, the step of extruding 300 the substrate to be fermented 12 can comprise subjecting the substrate to be fermented to a temperature higher than 40°C on the substrate to be fermented 12, preferably subjecting the substrate to be fermented to a temperature higher than 60°C on the substrate to be fermented 12, more preferably subjecting the substrate to be fermented to a temperature higher than 80°C on the substrate to be fermented 12 and event more preferably subjecting the substrate to be fermented to a temperature higher than 100°C on the substrate to be fermented 12. This heating can be due or mainly due to friction during the extrusion.

Advantageously, the step of extruding 300 the substrate to be fermented 12 is performed in order to keep a residence time of the substrate to be fermented 12 in the extruder of less than 10 minutes, preferably less than 5 minutes, more preferably less than 2 minutes. This residence time preferably corresponds to the time during which the substrate to be fermented 12 is in contact with the extrusion screw(s).

The step of extruding 300 the substrate to be fermented 12 can be carried out at a feed rate of at least 100 kg per hours of a substrate to be fermented 12, preferably at least 500 kg per hours of a substrate to be fermented 12, more preferably at least 1000 kg per hours of a substrate to be fermented 12 and even more preferably at least 2000 kg per hours of a substrate to be fermented 12.

The step of extruding 300 the substrate to be fermented 12 can be carried out with a screw(s) speed of at least 10 rpm, preferably at least 30 rpm, more preferably at least 50 rpm, even more preferably at least 100 rpm.

The cross section of the die of the extruder can take different shapes such as a parallelogram or an ellipse. The step of extruding 300 the substrate to be fermented 12 can be carried out with a die having one or more opening through which the substrate passes. The opening can have a cross section with an area value smaller than 2 cm², preferably smaller than 1 cm², more preferably smaller than 0.5 cm², even more preferably smaller than 0.2 cm². When considering the solid-state fermentation in vessels having an inner volume of 100 L or less, the applicant found that conducting an extrusion step of the substrate to be fermented through opening having a cross section with an area value smaller than 2 cm² allows an enhancement of the fermentation results. Such an extrusion step produces homogeneous structures that can be efficiently fermented.

The step of extruding 300 the substrate to be fermented 12 can be followed by a drying of the extruded substrate.

The extrusion 300 step can be configured to mix plant material from plants of different families. As it will be illustrated in example, mix of plants of different families can enhance the fermentation. Preferably, the extrusion 300 step can be configured to mix at least two plant material from plants of different families. Preferably, the extrusion 300 step can be configured to mix at least 10% in weigth of each of two plant material from plants of different families, more preferably it is configured to mix at least 20% in weigth of each of two plant material from plants of different families.

During the step of extruding 300 the substrate to be fermented 12, in particular in a preconditioner, water or steam can be added into the plant material or the substrate to achieve a desirable hydration of the substrate to be fermented 12 before feeding into the extruder. Alternatively, water or steam can be added into the plant material at extrusion screw(s) location.

As it will be illustrated in the example, the moisture content of the extruded substrate is preferably above 40%. The moisture content of the extruded substrate is more preferably of at least 50%, even more preferably of at least 60%. The moisture content of the extruded substrate is preferably under 100%. The moisture content of the extruded substrate is more preferably under 98%, even more preferably under 95%. However, the solid-state fermentation can include submerged fermentation. Preferably, the solid-state fermentation do not include submerged fermentation.

For example, the content of the substrate can be comprised between 40% and 95%, preferably range from 50% and 85%, more preferably range from 60% and 80%.

Such moisture content helps solid-state fermentation of the envisioned substrates as it helps to dissipate heat generated by the fermentation, and additionally, improves the gas exchange. It thus has a synergic effect with the use of small sized vessels as defined in the present invention, with the extrusion and with the moisture content.

The moisture is preferably analyzed using the protocole AOAC 930.15-1930(1999). Shortly, it comprises applying drying until reaching a stable weight of the sample. Moisture content is preferably determined using a moisture analyser arrange to dry and weight the substrate simultaneously.

A method according to the invention can comprise **a step of filling 400 vessels 42 with the extruded substrate to be fermented.** Preferably, the step of filling 400 vessels 42 with the extruded substrate to be fermented is a step of **automatic vessels** 42 filling 400 with the extruded substrate to be fermented.

A step of filling 400 vessels 42 with the extruded substrate to be fermented according to the invention can be implemented by an automated filling device 40 that will be described with the system according to the invention. Preferably, vessels 42 are moved by an automated system such as a conveyor system. The filling step is for example done, preferably continuously, at a rate of at least 50 vessels per hour, preferably at least 100 vessels per hours, more preferably at least 150 vessels per hour, and even more preferably at least 200 vessels per hour.

The vessels used in the present invention can be considered as small sized vessels. In particular, they have an inner volume of 100 L or less. Each vessel is preferably identical, having the same shape and same inside volume.

Use of small size vessels for solid-state fermentation has provided additional advantages over the known process. Because each reactor or vessel has a small volume (i.e. 100 L or less), each vessel used to produce a batch of fermentation product may be used with a loading factor of more than 30%. Furthermore, if any contamination of product happens, it will jeopardize only the content of a limited number of vessels. In addition, handling large volumes through numerous small units will also help to effectively achieve and maintain sterility.

According to the present invention, sterility only needs to be maintained inside each vessel (which is closed during fermentation time). Sterility is easy to maintain in small sized vessels, thanks to a closing of the vessel, said closing permitting aseptic, preferably axenic culture. For example, porous material as a filter or membrane that also permits aseptic, preferably axenic culture. The process according to the invention is also highly flexible. The controls of moisture, temperature, airflow, etc. may be made in different rooms or areas for different batches or products. The process according to the invention thus makes it possible to run different fermentations in parallel using different substrates, microorganism and environmental conditions, but using the same equipment.

Large-scale or industrial production means that the process and the production system according to the invention are configured to process several hundreds of kilograms of substrates per day. For example, the production system can be configured to process 500 kg of substrates to be fermented per day, i.e. to realize each step of the process according to the invention of such amount of substrate. For example, the production system can be configured to process 1 ton, 5 tons, 10 tons, or 15 tons of substrates per day. For example, if each vessel contains 500 g of the substrate to be cultured, this implies handling 1000 to 30 000 vessels per day. If each step of the process was carried out every day, a fermentation time of 10 days would thus imply that the production system comprised from 1 000 000 to 30 000 000 vessels.

The vessels used in the invention have an inner volume of 100 L or less, preferably 50 L or less, more preferably 20 L or less, and even more preferably 10 L or less. The inner volume of the vessel corresponds to its nominal capacity, i.e. to the maximum quantity (volume) of product that can be filled into the vessel. Small vessels, such as vessels having an inner volume of 100 L or less can facilitate automated manipulation and heat transfer.

A minimum inner volume of the vessel of 0.5 L can be advantageous. This corresponds to the minimum volume that is envisioned for an industrial production (smaller volumes could imply a very large number of vessels to be handled, and a minimum amount of substrate is preferred to perform the solid-state fermentation in good conditions). For example, a weight of more than 250 g (e.g. 350 g, 500 g,600 g, or 10 kg) of the substrate and inoculum per vessel is necessary to obtain a good yield.

Each of the vessels may have a maximum capacity comprised between 0.5 L and 100 L, more preferably between 0.5 L and 20 L and even more preferably between 0.5 L and 10 L. Use of small vessels is preferred for the above-mentioned reasons. This includes a possible use of vessels having a maximum capacity comprised between for example 0.5L or 1 L and 2 L, 5 L, 10 L, 15 L, 20 L, 30 L, 40 L and 50 L.

Vessels are filled with the extruded substrate. While the vessels are generally almost completely filled with substrate, air can remain inside each filled vessel, and air is present in the mass of introduced substrate.

According to a preferred embodiment of the invention, during the step of filling 400 vessels 42 with the extruded substrate to be fermented, each vessel 42 is filed with extruded substrate so that the ratio between the weight of the wet substrate (in kilograms) and the inner volume of the vessels (in Liters) is less than 1, preferably less than 0.9, more preferably less than 0.8 and even more preferably less than 0.7. Indeed, with such ratio, the fermentation of the extruded substrate in the closed vessels 42 is improved.

Optionally, the extruded substrate may be optimized after having been filled into the vessels, by perforation to form hole(s) in the substrate, or by shaking the filled vessel to accommodate the substrate.

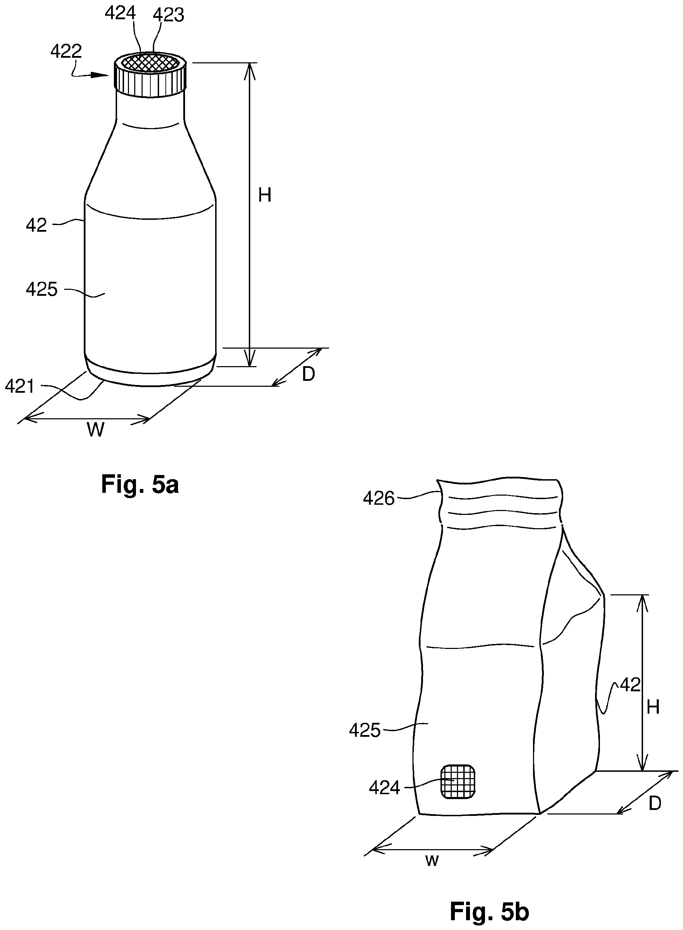

Preferably, the vessels 42 have three overall dimensions called height (H), width (W) and depth (D) measured in orthogonal directions, the smallest of the height (H), width (W) and depth (D) being less than or equal to 40 cm. Height, width and depth are measured in orthogonal directions. For a vessel conformed to rest on a base, on a horizontal surface such as on the ground, height is measured in the vertical direction, and corresponds to the dimension from the base of the vessel to its top (i.e. its higher point). Width corresponds to the greatest dimension of the vessel that is orthogonal to its height. Depth is the third dimension of the vessel, depth being measured in a direction orthogonal to height and width. Examples of the height H, the width W and the depth D of different vessels are shown in Figure 5a and Figure 5b.

More particularly, using vessels whose smallest dimension is less than or equal to 40 cm can enhance the gaseous homogeneity in the cultured substrates. For some applications, a smaller minimum dimension may be necessary, such as, 37 cm, 31 cm, 30 cm, 20 cm, or 10 cm. When at least one of these three dimensions is small (e.g. height H, width W or depth D of less than 40 cm), every point inside a vessel is situated at a short distance from a wall of the vessel (e.g. 20 cm at most), so that the heat transfer may occur from this point to the atmosphere through the wall of the vessel, and circulation of gas inside the vessel can be facilitated.

Gaseous flows naturally happen in the vessel (e.g. by natural convection), and also between the inside volume of the vessel and the atmosphere through barrier system such as a porous material, such as a porous membrane, preferably forming a microbial filter. An alcoholic fermentation is so avoided.

The porous material may in particular be carried by a cap used to close the vessel forming a barrier system of the vessels allowing to maintain it in a closed state. The porous material may be placed in other parts of the vessel. The inoculated vessel 52 may comprise one or several porous materials such as filters or membranes. The porous material allows gas exchange, but blocks contaminants such as spores, bacteria or other contaminants. For example, bacteria filtration is obtained with a membrane having a pore diameter of 1 µm or less.

No stirring of the extruded substrate having more than 40% of moisture content is necessary, and no forced air circulation is necessary to keep the cultured substrate in an adapted state for Solid-State Fermentation. The heat dissipation, aeration, humidity inside the vessel is efficient without any intervention required inside the vessels (such as. mixing, stirring, water addition, forced air, etc.) or outside the vessels (such as checking, vibration, cooling, etc.). Hence, thanks to the combination of vessels having an inner volume of 100 L or less, and of extruded substrate having more than 40% of moisture content, fermentation, at a level of fermentation observed in complex bioreactors with controlled growth, can be achieved.

Vessels according to the invention may have flexible or rigid walls. For example, flexible wall refers to a wall that can be deformed or have its shape changed like a fabric. In particular, a flexible wall is bendable and/or stretchable to follow the contour of the extruded substrate to be fermented and without irreversible change.

Preferably, vessels according to the invention have rigid walls. For example, rigid wall refers to a wall that cannot undergo mechanical stresses (e.g., bending or stretching) without significant irreversible change. Exemplary flexible wall includes wall made of glass, polymer, alloy, and/or metal.

The preferred vessels that can be used in the invention have the form of a bottle, a jar, a flask, or some form of tube or straight prism. In particular, the vessels used in the method of the invention may be for example bottles or bags. Bags generally refers to a vessel with floppy walls, which is deformed when it is filled. The above indicated maximum smallest dimension of the vessel used in the process refers in this case to the dimension of a fully filled bag.

The vessels are advantageously made of food-grade material such as polymers or alloy. For example, polysiloxane, polyethylene (high density or low density), polystyrene or polypropylene (also known as polypropene or poly (1-methylethylene)) may be used. Stainless steel may also be used.

As the invention requires a high number of vessels, thermosets or thermoplastics are materials providing an excellent compromise between cost, conductivity, food-grade properties, and resistance to a sterilization process.

Stainless steel can also be used. It has a high thermal conductivity, namely 14 to 14,5 W/mK. It is highly resistant and food grade. In terms of cost, it is more expensive than plastics, but it is more durable.

**Figure 5a and Figure 5b** represent example embodiments of vessels that may be used in a process or in a production system according to the invention.

Figure 5a shows a vessel having the form of a bottle. The bottle has a flat base 421 adapted to rest on a flat surface. Opposite to the base 421, the bottle has an open neck 422 that may be used to fill and empty the bottle. The size of the neck must thus be sufficient in particular to collect the cultured substrate after fermentation.

The height H of the bottle is defined as the dimension from the base 421 to the top of the neck 422. The represented bottle has a cylindrical general shape. The width W and the depth D of the bottle are thus the same, and are equal to the diameter of the bottle. The height H is, in the represented example, the greatest dimension of the bottle, and the width W and the depth D are the smallest dimensions of the bottle. They do not exceed 40 cm.

The bottle could, of course, have a different general shape. The smallest dimension could be its height. It could have any other prismatic general shape.

The bottle of Figure 5a comprises a cap 423. The cap 423 is provided with a filter 424, for example a microbial filtering porous membrane. The cap 423 may close the neck of the bottle and be attached to it according to several alternative known arrangements: it may for example be a screw cap, or it may be fitted to the neck by snap engagement. The interface between the cap and the neck is airtight, but the filter 424 allows gas exchanges between the inside and the outside of the vessel 42.

The walls 425 of the bottle may be formed in various food grade materials. It may be made of plastics, such as polyethylene (PE), polypropylene (PP), silicone, glass or stainless steel. Because the walls have to allow heat exchanges between the cultured substrate contained in the bottle and the atmosphere around the bottle, thin plastic walls or thin stainless steel walls are preferred.

Use of thermosets or thermoplastics is suitable for providing the required heat exchanges and provides an excellent compromise between cost, conductivity, food-grade properties, and resistance to a sterilization process.

Stainless steel has better thermal conductivity properties, is more durable, but is more expensive.

Figure 5b shows another embodiment of a vessel that may be used in a process or in a production system according to the invention. In the embodiment of Figure 5b, the vessel is formed of a bag. The vessel basically comprises a thin-walled pouch made of deformable plastic material. In the represented embodiment, once filled, the smallest dimension of the height (H); width (W) and depth (D) of the bag does not exceed 40 cm.

The walls of the bag can be formed of thin thermoplastics foils. As above explained the use of thermoplastics is suitable for providing the required heat exchanges and provides an excellent compromise between cost, conductivity, food-grade properties, and resistance to a sterilization process.

The bag comprises an opening 426 that is sealed (as shown in Figure 5b) after inoculation of the substrate to be cultured comprised of the bag. For example, the bag comprises a top part that is folded for closing the bag before sterilization. After sterilization, thermosealing may be used to seal the bag. Other known closing systems may be used, such as a sliding mechanism, or the bag may be provided with a port that can be closed by an adapted cap. A large opening facilitates emptying of the vessel.

In the represented embodiment, the wall 425 of the bag is provided with a filter 424, which allows gas exchanges between the inside and the outside of the vessel. In embodiments wherein the bag is closed by a cap, another filter may be provided on the cap 423 in alternative or complement to the filter provided on the wall of the bag.

A method according to the invention can comprise **a step of killing microorganisms 450.** The step of killing microorganisms 450 is done before the step of inoculating the microbial inoculant and thus before the solid-state fermentation occurs.

The step of killing microorganisms 450 can be applied to empty vessels 42, to vessels filed with the extruded substrate and/or to the substrate to be fermented 12 (extruded or not) before the filing of the vessels 42.

The step of killing microorganisms can be carried out by any means allowing elimination of microorganisms capable of damaging the substrate. In particular, the step of killing microorganisms can be a sterilization or a pasteurization.

Preferably, the step of killing microorganisms is carried out by a system, such as an automated system, capable of processing at least 10 vessels/h, preferably at least 100 vessels/h, more preferably at least 500 vessels/h.

For example, the step of killing microorganisms 450 is performed by batches of at least 10 vessels, preferably at least 100 vessels, more preferably at least 500 vessels.

The size of the batches used for the sterilizing step may depend on the size of a sterilization chamber where the sterilizing step is performed. Alternatively, continuous sterilization may also be considered. Preferably, the step of killing microorganisms is carried out by a continuous system capable of processing at least 10 vessels/h, preferably at least 100 vessels/h, more preferably at least 500 vessels/h.

The step of killing microorganisms is performed to inactivate microbes and their resistant structures (e.g.; spores). According to several embodiments of the invention, sterilization may be performed by using heat combined or not with high-pressure (e.g.; autoclaves, retorts, etc.) or by other alternative methods such as high temperature sterilization, low temperature sterilization, high-pressure sterilization, low-pressure sterilization, irradiation or chemical sterilization. The preferred type of sterilization for producing a fermentation product is a sterilization that can cause the destruction of all microorganisms in the substrate (whether or not pathogenic) and their spores, such as the heat combined with high-pressure sterilization.

After sterilization and if necessary cooling down, in an inoculating step, the substrate contained in the vessels is inoculated with a microbial inoculant. If the vessels have been air-tightly closed for sterilization, they may be stored in a certain time before inoculation.

A method according to the invention can comprise **a step of inoculating 500 the extruded substrate with a microbial inoculant.** Preferably, the step of inoculating 500 the extruded substrate with a microbial inoculant is a step of automated inoculation 500 of the extruded substrate with a microbial inoculant.

As already described, vessels can be handled and moved thank to automated systems. Preferably, the inoculation 500 step is executed by an automated inoculation station 50. Preferably the inoculation 500 step is executed under aseptic or hygienic conditions.

In particular, the microbial inoculant is adapted to cause a solid-state fermentation of the extruded substrate. A microbial inoculant adapted to cause a solid-state fermentation of the extruded substrate is for example a microbial inoculant comprising microbial strains which can grow on plant material in a solid-state fermentation.

The microbial inoculant used in the process may comprise one or more bacteria, yeast, filamentous fungi, and microalgae.

As previously indicated, the microbial inoculant may be one or more bacteria, yeast, filamentous fungi, and microalgae. Selection of the microorganism used in the microbial inoculant depends among other parameters on the final product sought, on its ability to develop in one or many type(s) of a given substrate, on its availability and price, etc.

Thus, the microbial inoculant used in the process may comprise one or more of bacteria (including actinomycetes), yeasts, and filamentous fungi; it is genetically modified or not. For example, one or several microorganisms of the below listed genera can be used in the invention.

### Bacteria & Actinomycetes

*Acetobacter, Actinomyces, Acinetocacter, Actinoplanes, Actinomadura, Aerococcus, Aeromonas, Alcaligenes, Alcanivorax, Alloiococcus, Alteromonas, Amycolatopsis, Anabaena, Arthrobacter, Arthrospira, Atopobium, Bacillus, Bifidobacterium, Brevibacterium, Brevundimonas, Carnobacterium, Catenisphaera, Cellulomonas, Citrobacter, Clostridium, Corynebacterium, Cyanobacteria, Dermatophilus, Desulfotomaculum, Dietzia, Enterobacter, Enterococcus, Escherichia, Frankia, Flavobacterium, Geobacillus, Gluconobacter, Gordonia, Humicola, Janthinobacterium, Lactobacillus, Lactococcus, Leuconostoc, Klebsiella, Marinobacter, Microbacterium, Micromonospora, Microtetraspora, Moraxella, Mycobacterium, Mycococcus, Micrococcus, Nocardia, Oenococcus, Pediococcus, Phormidium, Propionibacterium, Pseudomonas, Raoultella, Rastonia, Rhizobium, Rhodococcus, Saccharopolyspora, Serratia, Shigella, Sphingomonas, Staphylococcus, Streptococcus, Streptomyces, Symbiobacterium, Synechococcus, Synechocystis, Tetragenococcus, Thermoactinomyces, Thermomonospora, Vagococcuswhich, Vibrio, Weissella, Xanthomonas.*

Preferably, the microbial inoculant comprises bacteria which belong to sporulated bacteria and/or lactic bacteria. More preferably, the microbial inoculant comprises bacteria which belong to the taxonomic class Bacilli.

### Yeasts

*Achromobacter, Arxula, Aureobasidum, Blastobotrys, Brettanomyces (its perfect stage, Dekkera), Candida, Citeromyces, Cryptococcus, Cystofilobasidium, Debaryomyces, Endomycopsis, Filobasidiella, Galactomyces, Geotrichum, Glaciozyma, Guehomyces, Hansenula, Hanseniaspora (its asexual counterpart Kloeckera), Hyphopichia, Kluyveromyces, Kodamaea, Komagataella, Lachancea, Lipomyces, Metschnikowia, Moniella, Mrakia, Ogataea, Pichia, Phaffia, Pseudozyma, Rhodotorula, Rhodosporidium, Starmerella, Saccharomyces, Saccharomycodes, Saccharomycopsis, Scheffersomyces, Schizosaccharomyces, Schwanniomyces, Torulopsis, Torulaspora, Trichosporon, Trigonopsis, Yarrowia, Xanthophyllomyces and Zygosaccharomyces.*

Preferably, the microbial inoculant comprises yeast which belong to the genus *Brettanomyces* and/or *Aureobasidum.*

### Filamentous fungi

*Acremonium, Agaricus, Agrocybe, Akanthomyces, Alternaria, Ampelomyces, Amylosporus, Antrodia, Armillaria, Ashbya, Aspergillus, Atkinsonella, Aureobasidium, Auricularia, Balansia, Balansiopsis, Beauveria, Bispora, Bjerkandera, Boletus, Cantharellus, Catenaria, Cephalosporium, Chaetomium, Chrysonilia, Cladosporium, Claviceps, Clitocybe, Clitopilus, Colletotrichum, Collybia, Coniochaeta, Coprinus, Cordyceps, Coriolus, Cunninghamella, Cyathus, Cyclocybe, Cylindrocarpon, Cylinrocarpum, Cytonaema, Cytospora, Daldinia, Dentipellis, Doratomyces, Echinodothis, Emericella, Emericellopsis, Entoloma, Epichloe, Epicoccum, Favolaschia, Flammulina, Fomes, Fomitopsis, Fusarium, Ganoderma, Giberella, Gliocladium, Grifola, Gymnoascus, Hericium, Hohenbuehelia, Hormonema, Humicola, Hydropus, Hypomontagnella, Hypomyces, Hypoxylon, Hypsizigus, Inocutis, Inocybe, Inonotus, Isaria, Kuehneromyces, Lactarius, Laetiporus, Laxitextum, Lecanicillium, Lentinula, Lentinus, Lepista, Leptoshaeria, Lignosus, Lycoperdon, Lyophyllum, Martierella, Metarhizium, Monascus, Monilia, Monocillium, Morchella, Mortierella, Mucor, Mycelia, Myriogenospora, Neurospora, Nigrospora, Omphalotus, Ophiocordyceps, Oudemansiella, Paecilomyces, Panellus, Panus, Paraconiothyrium, Paraepichloe, Penicillium, Peniophora, Periconia, Pestalotiopsis, Phellinus, Phlebia, Pholiota, Phoma, Phomopsis, Piptoporus, Pleurotus, Pochonia, Polyporus, Preussia, Pycnoporus, Ramaria, Rhizopus, Rhodotus, Sarcodon, Schizophyllum, Scytalidium, Scytalidium, Scytinostroma, Sparassis, Spicaria, Stachybotrys, Steccherinum, Stropharia, Suillus, Thermoascus, Thermomyces, Tolypocladium, Torula, Trametes, Tremella, Trichoderma, Tricholoma, Tuber, Verticillium, Volvariella, Wolfiporia, Wrightoporia, Xylaria.*

Preferably, the microbial inoculant comprises at least one microorganism known to have lignolytic or cellulolytic activity. Indeed, the applicant found that the method of the invention gives particularly interesting results with such strains.

More preferably, the microbial inoculant comprises at least one Dikarya fungus. Indeed, the applicant found that the method of the invention gives particularly interesting results with such strains. The Dikarya fungi belong to a fungal phylum selected from: *Basidiomycota* or *Ascomycota.*

Even more preferably, the Dikarya fungi is a white-rot fungus or a brown-rot fungus, and can be selected from *Pleurotus* sp, *Morchella* sp., *Laeitoporus* sp, *Flammulina* sp and *Fomitopsis sp.*

In an embodiment, the microbial inoculant comprises at least one Dikarya fungus in combination with one or more species of bacteria, yeasts, fungi and/or microalgae.

As illustrated in **figure 3****,** the inoculation 500 step can comprise following sub steps: opening 510 the vessels; seeding 520 the microbial inoculant in the vessels; and/or closing 530 the vessels.

Alternatively, the inoculation 500 step can comprise a step of seeding 520 the microbial inoculant on the extruded substrate throught a vessel wall, for example through a polymer septum or through the barrier system (e.g. through a cap).

In particular, the inoculation can comprise: opening the vessels 42, implanting the microbial inoculant such as spores, mycelia, cells, etc. on the extruded substrate, and closing the vessels 42 again. As for filling the vessels, this step comprising multiple but simple operations, which have to be carried out for each vessel 42 is preferably performed by automated means. This step is performed in aseptic conditions, to avoid any contamination of the substrate during inoculation. Alternatively, the vessels 42 may be kept open for sterilization and inoculation, and be closed only after inoculation. After the inoculating step, the inoculated vessels 42 are in a closed state. For example, a cap is placed on a neck of the vessel, or another closing system such as a sliding mechanism to seal a bag, or other sealing mechanism.

Preferably, the vessels 42 are in a closed state after the inoculating step 500. The closed state is arranged to allow gaseous exchanges between the inside of the inoculated vessels 52 and the outside.

For example, the gaseous exchanges between the inside of the inoculated vessels 52 and the outside can be controlled by barrier system such as a device comprising a porous material 423, 424 preventing microorganisms from entering the vessel.

Preferably, the barrier system including or not a cap 423, can comprise a porous material, such as a filter 424, having pore size 1 µm or lower, 0.45 or lower, more preferably 0.22 µm or lower.

Hence, the vessels, in the closed state, can be water-tightly sealed. However, gas exchanges with the atmosphere surrounding the vessel must be possible during fermentation. For that purpose, each vessel has one or several porous materials (e.g. at the level of the cap used to close the vessel after implantation of the microbial inoculant) that allow gas exchanges, but block contaminants such as spores, bacteria or other contaminants. A porous membrane may be used, more particularly a bacteria filtering hydrophobic porous membrane.

A method according to the invention can comprise **a step of storing 600 the inoculated vessels 52.** In particular, the inoculated vessels 52 are stored in a closed state, in a controlled climate area 60. Storing in open state is possible but necessitate aseptic conditions for the storage which is not efficient in large-scale fermentation. Moreover it can become complicated in the case of a contamination of a bottle because it will be a risk of dispersion of the contamination.

Preferably, the inoculated vessels 52 are stored in controlled climate conditions. The temperature in the controlled climate area 60 is controlled to promote the development of the microorganism in the cultured substrate and fermentation. A temperature of 10°C to 50°C is generally appropriate (depending on the substrate and on the microbial inoculant). Other controlled parameters in the controlled climate area 60 may be hygrometry, oxygen and CO₂ levels. For example, the humidity is maintained above 35%, preferably above 45%, more preferably above 50%, even more preferably above 60%, for example above 65%. CO₂ levels can be maintained between 600 and 1200 ppm, preferably 800 and 1000 ppm.

Generally, the inoculated vessels 52 are stored during a time sufficient for a fermentation of the extruded substrate. The fermentation may not be completed but at least a part of the content of the vessels (i.e. the extrudate substrate) should be fermented. The time sufficient for a fermentation of the extruded substrate can be for example at least one day, preferably at least 5 days, more preferably at least 10 days and even more preferably at least 15 days, for example at least 20 days. The time sufficient for a fermentation of the extruded substrate can be for example at most 60 days, preferably at most 50 days, more preferably at most 40 days and even more preferably at most 30 days. The person skill in the art will be able to choose time sufficient for a fermentation depending on the microbial inoculant and the growth conditions.

In particular, the inoculated vessels 52 are stored in a closed state during a time sufficient to observe weight loss, preferably weight loss higher than 1%. Indeed, the loss of weight can be considered as a marker of the fermentation of the extruded substrate.

Solid-state fermentation happens during the storing 600 step. This step can carry on until colonization of the cultured substrate is complete (or almost complete). By way of example, the storing step may last from 1 to 60 days, depending on the volume of the vessel, the climate conditions, the cultured substrate and the microbial inoculant. The combination of extruded substrate with a controlled moisture content and small size vessels allows an efficient fermentation without mixing forced aeration humidification devices and heat dissipation partition plates.

A method according to the invention can comprise **a step of harvesting 700 the content of the inoculated vessels 52.** Preferably, the method according to the invention comprises a the step of automatic harvesting 700 of the content of the inoculated vessels 52. Moreover, this step comprises an opening, preferably an automatic opening, of the vessels just before the harvesting. In particular, the vessels can be openend less than three hours before the harvesting, preferably less than one hour, more preferably less than five minutes, and even more preferably less than one minute before the harvesting.

Preferably, the step of harvesting 700 comprises harvesting at least a part of the content of the inoculated vessels 52. Preferably, harvesting 700 step comprises collecting at least 30% in weight of the content of the inoculated vessel 42, more preferably at least 50% in weight, and even more preferably at least 80% in weight.

The content of the inoculated vessels 52 will generally consist of the fermented substrate and microbial biomass. For example, when the microbial inoculant comprises at least one bacilli, the content of the vessels to be harvested will comprise the fermented substrate and/or vegetative cell and/or bacilli spores. For example, when the microbial inoculant comprises at least one Dikarya fungus, the content of the vessels to be harvested will comprise the fermented substrate and/or fungal mycelium and/or spores.

In particular, after colonization, the vessels are opened, emptied, and the fermentation product is collected. Harvesting is generally performed under aseptic conditions, to avoid contamination of the fermentation product. In particular, an automated harvesting device 70 can be configured to open the inoculated vessels 52 and to collect the fermented substrate and the microbial biomass, preferably under aseptic conditions.

The vessels can be opened through a cap removal, the opening of a sliding mechanism or thanks to a tear made in the vessels.

Preferably, harvesting 700 step is carried out by an automated harvesting device 70 configured to collect at least 30% of the content of the vessel, preferably under aseptic conditions.

A fermentation product is so obtained, in large quantity, thanks to a process using multiple simple fermentation vessels instead of a large and complex bioreactor.

A method 1 according to the invention can comprise **a step of processing 800 the harvested content.**

This step of processing can be designed to obtain a fermentation product from all or part of the content of the inoculated vessels 52.

The fermentation product can be selected from: liquid preparations such as beverage, gel/paste preparations, or powders preparations such as flours. Moreover, the fermentation product can comprise content selected from: spores, enzymes, organic acids, fatty acids, carboxylic acids, amino acids, phenolic acids, other organic acids, other metabolites (e.g. esters, sugars, sugar-alcohol, polyols, etc), nucleotides, vitamins, polyphenols, proteins, antioxidants, antimicrobials (e.g. antibiotics), fibres, fats, pigments, dyes, hormones, flavours, fragances, odorants, gelification agents, acidulants, antivirus agents, pheromones, biomaterials, polymers, biosurfactants, and microbial biomass..

Preferably, the fermentation product comprises: spores, enzymes, pigments, flavours, fragances, microbial biomass, and/or antimicrobials. More preferably, the fermentation product comprises: spores, enzymes, microbial biomass, and/or antimicrobials. Even more preferably, the fermentation product comprises: spores, microbial biomass, and/or antimicrobials.

For example, the fermentation product (i.e. obtain throught the steps of the fmermentation process according to the invention including post fermentation steps), comprises at least 5% in weight of: spores, enzymes, pigments, flavours, fragances, microbial biomass, and/or antimicrobials; preferably at least 10% in cumulated weight; more preferably at least 20% in cumulated weight; even more preferably at least 20% in cumulated weight.

Preferably, the step of processing is designed to obtain a fermented edible product. The fermented edible product can for example be fermented flour or comprises probiotics.

As illustrated in the **figure 4****,** the step of processing 800 can comprise optional drying 810 of the fermented substrate; optional grinding or milling 820 of the fermented substrate and/or optional packing 830 of the fermented substrate.

For example, the harvesting step may be followed by a step of drying the fermentation product. For example, the fermented dried product may be ground to form a flour. The obtained fermented flour may be of high nutritional interest. It has generally a low sugar content, and has a high fiber content. It may be gluten free. The steps of drying and grinding may be performed simultaneously.

Alternatively, the harvesting step may be followed by a packing step. The fermentation product being a raw mix of fermented substrate and microbial biomass.

Advantageously, the emptied vessels are cleaned for reuse, i.e. to be sent to the step of filling vessels with extruded substrate of a further same process.

In another aspect, the invention relates to a **system 2 for solid-state fermentation of plant material.**

In particular, the system 2 for solid-state fermentation of plant material can be used to perform a method for solid-state fermentation of plant material according to the invention. Hence, the system 2 for solid-state fermentation of plant material according to the invention is arranged and/or configured to implement all embodiment of the method according to the invention. In particular, the system 2 for solid-state fermentation of plant material according to the invention is arranged and/or configured to implement the preferred and advantageous embodiment of the method according to the invention.

In particular, the system 2 for solid-state fermentation of plant material can be used to produce a fermentation product, preferably a fermented edible product, said fermented edible product comprising fermented plant material.

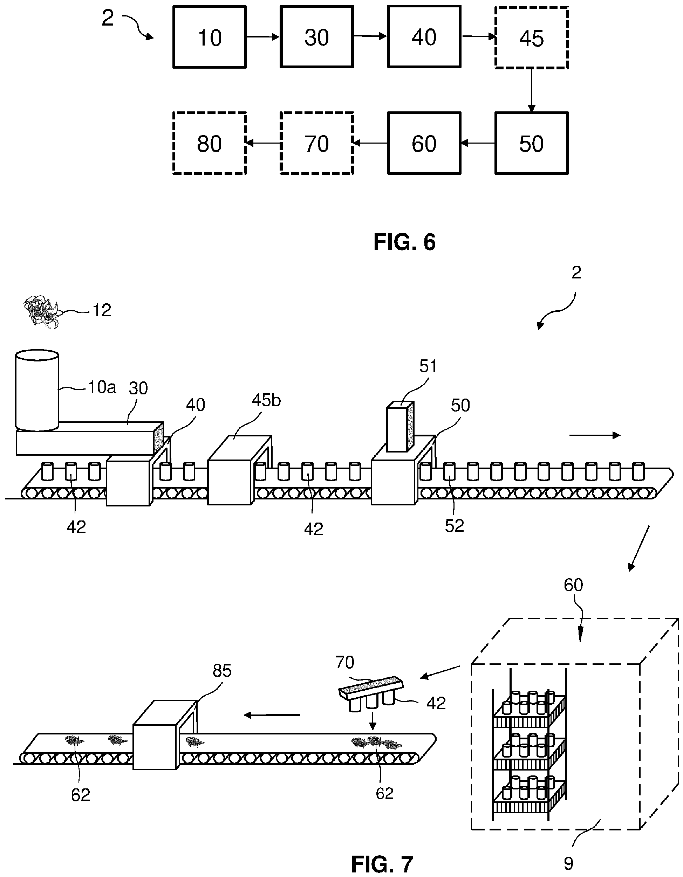

As illustrated in the **figure 6****,** the system 2 for solid-state fermentation according to the invention comprises: a supply device 10, an extruder 30, an automated filling device 40, an automatic inoculating device 50, and an incubation chamber 60.

As illustrated in the figure 6, the system 2 for solid-state fermentation according to the invention can also comprises: a device adapted to kill microorganisms 45, an automated harvesting device 70 and an automated processing device 80.

Moreover, the system 2 for solid-state fermentation advantageously comprises an automatic conveying system such as a conveyor belt. This is a well-known and convenient automatic conveying system. Other automatic conveying systems may be used in the production system as a complement or alternative to conveyor belts. For example, automated guided vehicles may be used. The vehicle can implement various automation technologies for its guidance, which may be accomplished by wire guidance, optoguidance, and / or by more flexible technologies based on geolocation of the vehicle.

The supply device 10 is preferably arranged to receive a substrate to be fermented 12. As it has been detailed the substrate to be fermented comprises plant material. The supply device can for example be a supply hopper. Alternatively, it can be an automated supply device such as an endless screw. The supply device 10 is more preferably configured to provide a substrate to be fermented 12 at least 100 kg per hours.

**The extruder 30** is preferably arranged to extrude the substrate to be fermented 12 at a moisture content higher than 40%. More preferably, the extruder 30 is arranged to extrude the substrate to be fermented 12 at a predetermined moisture content that is higher than 50%.

An extruder according to the invention includes generally the following parts : motor, barrel, screw(s), and die(s). It can also include one of several cutter(s). Along with these parts, there may be many other parts or pieces of equipment either upstream or downstream that help with either the preparation of the substrate mixes before extrusion or post-processing of the extruded products after extrusion. Extruders typically include one or two screws, encased in a barrel that is either grooved or smooth. The screw(s) is driven by a motor, typically with a gearbox that can help with varying screw speeds during its operation. In addition, most often there is a die at the end of the barrel, through which the material is forced out of the extruder.

An extruder can be divided into several distinct sections such as conveying, compression/mixing, and metering/melting sections. The screw profile can be adapted to accomplish the specific functions in each of these sections.

The conveying section is designed mainly to convey the material continuously to the compression section in the extruder. In this section, the screw elements are often designed to have a large pitch with large volume so that the material is filled and conveyed to the next section.

The compression section is where the material is compacted and mixed simultaneously. In this section, the screw elements are short pitched to facilitate the compaction. Compaction along with the movement of the materials against each other leads to the generation of heat, because of the shear between plant material particles and between plant material particles and the screw and/or barrel surface.

The final section metering/melting is where the material generally transforms into its melt state, due to the heat generated from the mechanical shear and the pressure. In this section, the pressure is usually high due to the compaction of the material and the restriction imparted by the die which is located at the end of the barrel section.

An extruder used in a system according to the invention can comprise at least a section of conveying and a section of compression/mixing.

Die helps to shape the final product. The die can cause pressure to build up in the zone behind the die. This pressure has a significant impact on the shear generated in the last zone of the extruder. Following the die, the system typically has a cutter setup.

The automated filling device 40 is preferably arranged to fill vessels 42 with the extruded substrate. As it has been detailed vessels 42 have an inner volume of 100 L or less. Hence, the automated filling device 40 is preferably arranged to fill vessels having an inner volume of 100 L or less. The automated filling device 40 can be, or can comprise, a bottler when the vessels are bottles.

For example, the automated filling device 40 is further arranged to fill vessels with the extruded substrate to at most 90% of their volume, preferably at most 70% of their volume. In an advantageous embodiment, the system 2, for example the automated filling device 40 is configured to perform one or several hole(s) in the extruded substrate to improve gas and heat exchange. For example, said hole can have a diameter of one cm or more, preferably a diameter of two cm or more.

The system can comprise the vessels 42. As described, preferably, each inoculated vessel 52 in the closed state comprises a barrier system 424 made of a porous material such as a porous membrane allowing gaseous exchanges between an internal volume of the inoculated vessel 42 and the controlled climate area 60. The porous membrane has preferably pores of 1 µm or less.

The device adapted to kill microorganisms 45 can be sterilizer or a pasteurizer. It can use radiation or heat treatment.

The automatic inoculating device 50 is preferably configured to inoculate the extruded substrate with a microbial inoculant. As it has been described the microbial inoculant is adapted to cause a solid-state fermentation of the extruded substrate. The automatic inoculating device 50 can be further arranged to seed the extruded substrate in depth, with a microbial inoculant contained in a microbial inoculant storage 51. Preferably, the automatic inoculating device 50 is arranged to seed at least part of the microbial inoculant in the middle of the vessel, preferably the middle height of the vessel. More preferably, the automatic inoculating device 50 is arranged to seed at least part of the microbial inoculant in several holes made in the extruded substrate.

The incubation chamber 60 is preferably arranged to allow a solid-state fermentation of the extruded substrate by the microbial inoculant. This solid-state fermentation is conducted in the inoculated vessels 52 that are being in a closed state after the inoculating step. The incubation chamber 60 is preferably configured to maintain growth conditions corresponding to the preferred growth conditions of the process according to the invention.

The automated harvesting device 70 is preferably arranged to harvest the content of the inoculated vessels 52 after a growth period. After the growth period, the inoculated vessels 52 comprises the fermented substrate 62 and microbial biomass from the microbial inoculant. Hence, the automated harvesting device 70 is preferably configured to collect at least part of the fermented substrate. For example, the automated harvesting device 70 is configured to collect at least 50% in weight the fermented substrate, preferably at least 70% in weight the fermented substrate, more preferably at least 90% in weight the fermented substrate, even more preferably at least 95% in weight the fermented substrate. Indeed, the fermented substrate will part of the value of the fermentation product.

The automated processing device 80 can be configured to process the harvested content to obtain a fermentation product from all or part of the content of the inoculated vessels 52. The automated processing device 80 can preferably be configured to produce a fermentation product, for example a fermented edible product, more preferably a fermented flour, from all or part of the content of the inoculated vessels 52.

The automated processing device 80 can be configured or coupled with equipment to dry, grind, mill or pack the fermented substrate. Automated processing device 80 can comprise a dryer 85 and at least one of a grinder or a mill. The dryer and the grinding machine may, in some embodiments, be formed by a single machine such as a rotor pulverizer and dryer.

**Figure 7** schematically represents a system 2 for solid-state fermentation of plant material according to an embodiment of the invention.

The system 2 corresponds to a set of machines and equipment, installed in one or several buildings, used to perform a method for solid-state fermentation of plant material according to the invention.

The system 2 may comprise one or several items of equipment for substrate preparation, such as a crusher, a boiler, a steamer, a washer, a presser, and/or a mixer.

The system also comprise a hopper 10a, or a similar item, for the collection of the prepared (or directly provided) substrate to be fermented. The substrate to be cultured is distributed from the hopper 10a to an extruder 30. The extruder 30 is preferably configured to extrude the substrate to be fermented at a predetermined value of moisture content, more preferably a value higher than 40%. The extruded substrate is distributed to the automatic filling system 40.

The automatic filling system 40 is adapted to fill vessels 42 with a predefined quantity (volume or mass) of extruded substrate. The automatic filling system 40 may also adapt to close, for example to cap, the filled vessels 42. If the vessels 42 are bottles, the automatic filling system 40 may comprise or may be a bottler. A linear system is advantageously used instead of a bottling carousel.

The automatic filling system 40 may be formed of two distinct machines respectively for filling the vessels 42 and for closing the vessels.

The vessels 42 are advantageously provided to the automatic filling system 40 by an automatic conveying system such as a conveyor belt. The filed vessels can go through a sterilization device 45b.

Alternatively, the filled vessels can be sent to a sterilization chamber, for sterilization of the vessels and of their content. For this operation, in particular if a sterilization chamber is used, the filled and closed vessels may be grouped in batches, e.g. they may be placed on pallets or suitable crates to be manually or automatically transported into the sterilization chamber. Manual transport corresponds to a transport using for example a human guided manual or electric pallet truck.

While the represented production system comprises a sterilization chamber, other systems performing a continuous sterilization such as continuous sterilization retorts may be used. In such case, the vessels may be placed, after sterilization, on pallets or suitable crates to be manually or automatically transported.

After sterilization, the (sterilized) substrate to be cultured is inoculated with a microbial inoculant. This step, comprising at least the operations of opening each vessel, implanting the microbial inoculant(s) on the substrate, and closing the vessels, is preferably performed in an automatic inoculating device 50. The vessels are preferably transported (e.g. de-palletized and carried) to the automatic inoculating device 50 by an automatic conveying system. Opening, implanting the microbial inoculant and closing the vessels is performed in aseptic conditions inside the automatic inoculating device 50. Alternatively, the vessels can be inoculated through a membrane, such as an elastic membrane, allowing an aseptic inoculation.

To optimize the production process, one or several buffers may be provided, where vessels can be stored between two steps of the process. For example, filed vessels may be stored before and/or after the sterilization step. In particular, the filed vessels may be stored after the sterilization step for several days or weeks. This may improve operation efficiency and be especially useful in the year-round treatment of seasonal supply raw materials/substrates

After the sterilization step, provided that the vessels are closed, the vessels may be stored before the inoculating step. It should be noted that in such case, the caps (for example) used to close the vessels for sterilization and further storing may be different (e.g. provide an airtight closure) from the caps used to close the vessels after inoculation (to allow gas exchanges).

The production system further comprises a controlled climate area 60. The controlled climate area 60 is an area which is adapted to storing the vessels, e.g. in grouped and/or palletized form for example in racks or shelves, and where the environmental parameters are controlled.

The controlled parameters may comprise temperature, hygrometry, oxygen level and carbon dioxide level, and light or darkness. Sensors may be installed in the controlled climate area 60 to measure one or several environmental parameters, and corrections means may be used if any parameter drifts outside a predefined value range. The controlled climate area 60 may be provided with a heater and/or an air conditioner, an air desiccator or a fogger, a ventilation system, etc.

Of course, several controlled climate areas may be arranged in the production system. This makes it possible to carry out several fermentation processes in parallel, with, if needed, different environmental parameters.

An automatic emptying system may be provided, for emptying the vessels of their content after fermentation.

The automatic emptying system allows harvesting of the fermentation product under at least hygienic conditions, for fermented food products. For other products, such as some pharmaceutical products or a biochemistry products, harvesting may be performed under sterile conditions.

After harvesting, a large quantity of fermentation product is obtained, for example in a fermentation product receptor.

The emptied, used, vessels may be then cleaned, for example in a vessel cleaning machine such as a cleaning tunnel. The cleaned vessels may then be reused.

The represented production system is a system for producing an edible fermented product. The fermented product can be provided to a dryer, where it is dried. The dried fermented substrate is ground in a grinding machine.

A fermented flour is obtained and packed.

### Example

### I. Comparison between bioreactor and vessels

### Example 1:

400 kg of an extruded substrate having a moisture content of 70% is prepared from apple by-product with an initial moisture of 10%.

A 400 liters bioreactor is manualy filed with 200 kg of extruded substrate to be fermented.

400 vessels having a 1 L inner volume are automatically filled each with 500 g of extruded substrate and microbial inoculant (*Morchella* sp).

Fermentation lasted from 20 to 40 days, with the following stable conditions: incubation: static; Room conditions: Temperature: 25°C; Humidity: above 65%; CO₂: Between 800 and 1000 ppm, without forced ventilation, no stirring and no control temperature inside the vessels or the bioreactor.

The content of the bioreactor and the content of the vessels is automatically harvested under hygienic conditions and analyzed.

The extruded substrate in the vessels is mostly colonized after incubation time whereas the extruded substrate in the bioreactor is only partially colonized.

A combination of extrusion of the substrate, moisture content control and vessels use allow an efficient solid-state fermentation of plant material compared to the use of a large bioreactor.

Moreover, the contaminated vessels, for example can be identified by visual inspection, can be removed without much impact in the production yield whereas a contamination in the 200L bioreactor can be disastrous for production yield.

### II. Fermentation product preparation

### Example 2: Fermented edible flour

Apple by-product, peels, at 10% moisture was used as plant material for the preparation of a substrate to be fermented.

Apple by-product was whether extruded or not for the preparation of a substrate having a moisture content of 70%, 60% or 40%.

Vessels having a 1 L inner volume were automaticaly filled each with 500 g of substrate (extruded or not) and microbial inoculant *(Pleurotus* sp.).

Fermentation lasted from 10 to 40 days, with the following stable conditions: incubation: static; Room conditions: Temperature: 25°C; Humidity: above 65%; CO2: Between 800 and 1500 ppm without forced ventilation, no stirring and no control temperature inside the vessels.

The content of the vessels was automatically harvested under hygienic conditions to obtain a fermentation product, in particular a fermented edible product.

This fermented edible product is further dried and milled in order to produce a fermented edible flour.

### Example 3 : Pet food preparation

Sugar beet by-product at 10% moisture was used as plant material for the preparation of a substrate to be fermented.

100 kg of sugar beet by-product was either extruded or not for the preparation of a substrate having a moisture content of 70%.

200 vessels, having a 2 L inner volume, were automatically filled each with 500 g of substrate (extruded or not) and microbial inoculant *(Pleurotus* sp.).

Fermentation lasted 30 days, with the following stable conditions: incubation: static; Room conditions: Temperature: 25°C; Humidity: above 65%; CO₂: between 800 and 1000 ppm, without forced ventilation, no stirring and no control temperature inside the vessels.

The content of the vessels was automatically harvested under hygienic conditions to obtain a fermentation product. The fermentation product can be further extruded with cereals, meat broth and water to obtain a pet food.

### Example 4 : Meat substitute

Orange by-product at 10% moisture was used as plant material for the preparation of a substrate to be fermented.

Orange by-product was whether extruded or not for the preparation of a substrate having a moisture content of 70% or 65%.

Vessels having a 2 L inner volume were automatically filled each with 500 g of substrate (extruded or not) and microbial inoculant *(Pleurotus* sp.).

Fermentation lasted 30 days, with the following stable conditions: incubation: static; Room conditions: Temperature: 25°C; Humidity: above 65%; CO₂: Between 800 and 1500 ppm, without forced ventilation, no stirring and no control temperature inside the vessels.

The content of the vessels was automatically harvested under hygienic conditions to obtain a fermented edible product. The fermented edible product can be further mixed with vegetables to obtain a meat substitute.

### Example 5: fermented edible product

Apple pomace at 10% moisture was used as plant material for the preparation of a substrate to be fermented.

200 kg of apple pomace was whether extruded or not for the preparation of a substrate having a moisture content of 70%.

400 vessels having a 2 L inner volume were automatically filled each with 500 g of substrate (extruded or not) and microbial inoculant *(Pleurotus* sp.).

Fermentation lasted 30 days, with the following stable conditions: incubation: static; Room conditions: Temperature: 25°C; Humidity: above 65%; CO₂: between 800 and 1500 ppm, without forced ventilation, no stirring and no control temperature inside the vessels.

The content of the vessels was automatically harvested under hygienic conditions to obtain a fermented edible product.

### Example 6: Spores production

Orange by-product and wheat bran at 12% and 10% moisture respectively were used as plant materials for the preparation of a substrate to be fermented. The ratio Orange: Wheat bran being of 1:1.

The mix was extruded having a moisture content of 60%.

Vessels having a 50 L inner volume were automatically filled each with 12,5kg of substrate and microbial inoculant (*Aspergillus* sp).

Fermentation lasted 15 days, with the following stable conditions: incubation: static; Room conditions: Temperature: 25°C; Humidity: above 65%; CO₂: between 800 and 4000 ppm, without forced ventilation, no stirring and no control temperature inside the vessels.

The content of the vessels was automatically harvested under hygienic conditions and processed to obtain a mass spores of *Aspergillus* sp.

The spores of *Aspergillus* sp can produce gluconic acid that can be used in meat, dairy products, particularly in baked goods as a component of leavening agent, generally in food product as a flavouring agent or also as mineral supplements to prevent the deficiency of calcium, iron etc.

### Example 7: Enzyme production

Orange by-product and corn cobs at 12% and 9% moisture respectively were used as plant materials for the preparation of a substrate to be fermented. The ratio Orange: corn cobs was 1:0.2. The mix was extruded having a moisture content of 70%.

Another composition is used with corn cobs at 9% moisture as plant materials for the preparation of a substrate to be fermented. The composition is extruded having a moisture content of 70%.

Vessels having a 5 L inner volume were automatically filled each with 3Kg of substrate and microbial inoculant (*Geotrichum* sp).

Fermentation lasted 10 days, with the following stable conditions: incubation: static; Room conditions: Temperature: 28°C; Humidity: above 65%; CO₂: between 500 and 1000 ppm, without forced ventilation, no stirring and no control temperature inside the vessels.

The content of the vessels was automatically harvested under hygienic conditions and processed to obtain proteolytic enzymes product.

The substrate based solely on corn cobs is significantly less fermented than the mix of Orange by-product and corn cobs. The use of vegetables and/or fruits by-products, alone or combined with other plant material in the extrudate substrate improve the fermentation. In addition, these substrates can offer richer nutritional profile and an efficient fermentation.

### Example 8: Probiotics

Carrot by-product at 90% moisture was used as plant material for the preparation of a substrate to be fermented.

The mix was extruded having a moisture content of 80%.

Vessels having a 1 L inner volume were automatically filled each with 500 g of substrate and microbial inoculant (*Bacillus* sp).

Fermentation lasted 2 days, with the following stable conditions: incubation: static; Room conditions: Temperature: 35°C; Humidity: above 65%; CO₂: between 400 and 1000 ppm, without forced ventilation, no stirring and no control temperature inside the vessels.

The content of the vessels was automatically harvested under hygienic conditions to obtain a mass of *Bacillus* sp. spores.

The *Bacillus* sp. spores can be dried and used in several industrial processes or agri-food processes. In particular, *Bacillus* sp. spores can be added to food product as health beneficial probiotics.

### Example 9: Flour

Tomato by-product at 75% moisture was used as plant material for the preparation of a substrate to be fermented.

The mix was extruded having a moisture content of 75%.

Vessels having a 0.8 L inner volume were automatically filled each with 500 g of substrate and microbial inoculant (*Laetiporus* sp).

Fermentation lasted 20 days, with the following stable conditions: incubation: static; Room conditions: Temperature: 28°C; Humidity: above 80%; CO₂: between 800 and 1000 ppm, without forced ventilation, no stirring and no control temperature inside the vessels.

The content of the vessels was automatically harvested under hygienic conditions to obtain a fermented edible flour.

### Example 10: Composition comprising proteolytic enzyme

Grape marc at 55% moisture was used as substrate, with an Ascomycetes inoculum.

Vessels having a 2 L inner volume were automatically filled each with 600 g of extruded substrate and inoculum.

Fermentation lasted 10 days, with the following stable conditions: incubation: static; Room conditions: Temperature: 28°C; Humidity: above 65%; CO₂: between 800 and 1000 ppm, without forced ventilation, no stirring and no control temperature inside the vessels.

The content of the vessels was automatically harvested under hygienic conditions to obtain a fermented edible product comprising proteolytic enzymes.

### Example 11: Mix of plant material

Grape marc at 55% moisture was used as substrate, with an Ascomycetes inoculum. Potatoes, at 55% moisture, were used as a co-substrate.

Vessels having a 2 L inner volume were automatically filled each with 1600 g of extruded substrate and inoculum or with extruded substrate/co-substrate and inoculum. Various quantities of potatoes were added to the Grape marc.

Fermentation lasted 20 days, with the following stable conditions: incubation: static; Room conditions: Temperature: 28°C; Humidity: above 65%; CO₂: between 800 and 2000 ppm, without forced ventilation, no stirring and no control temperature inside the vessels.

The content of the vessels was automatically harvested under hygienic conditions to obtain a fermented edible product.

### III. Enhancement of the fermentation by the extrusion

The following table 1 illustrates how the extrusion step can enhance the solid-state fermentation (SSF) of several substrates comprising plant material when the SSF is conducted in vessels. The weight loss of the vessels content was measured during fermentation and at the harvesting time. In the table 1 is reported the percent of weight loss depending on the extrusion or not of the substrate at the harvesting time. For some substrates, the extrusion was combined with a grinding of the substrate before the extrusion.

**Table 1**

| | **Unextruded** | **Extruded** | **Grinded - Extruded** | **Fermentation enhancement from extrusion** |
|---|---|---|---|---|
| Ex2 Apple peels; moisture content 70% | 5.24% | 6.79% | / | +29.6% |
| Ex3 Sugar beet; moisture content 70% | 5.78% | 7.76% | 7.03% | +34.25% |
| Ex4 Orange; moisture content 70% | 6.08% | 6.87% | / | +13% |
| Ex4 Orange; moisture content 65% | 6.64% | 8.96% | / | +35% |
| Ex5 Apple pomace 70% | 2.23% | 4.39% | 4.28% | +97% |

Table 1 illustrates that the extrusion can enhance the fermentation of all the tested substrate from +13% (orange at 70% of moisture content) to +97% (apple pomace at 70% of moisture content).

Table 1 also shows that a grinding step before the extrusion is not of particular use to enhance the weight loss during fermentation.

Hence, when the plant material is fermented by solid-state fermentation in vessels in a method according to the invention, the extrusion is of particular use.

### IV. Impact of the moisture content of the extruded substrate

The following table 2 illustrates how the moisture content of the extruded substrate can impact the solid-state fermentation (SSF) of two substrates comprising plant material when the SSF is conducted in vessels. The weight loss of the vessels content was measured during fermentation and at the harvesting time. In the table 2 is reported the enhancement of weight loss compared to unextruded substrate.

**Table 2**

| | **Extruded 40%** | **Extruded 45%** | **Extruded 60%** | **Extruded 65%** | **Extruded 70%** | **Extruded 80%** |
|---|---|---|---|---|---|---|
| Ex2: Apple peels | No growth observed | / | +5.1% | / | +29.6% | / |
| Ex4 : Orange | No growth observed | / | / | +35% | +13% | / |
| Ex6: Orange | No enhancement by extrusion | +6.8% | +18% | / | / | / |
| Ex8: Carrot | No growth observed | / | / | / | +21.4% | +25.6% |

Table 2 illustrates that the moisture content of the extruded substrate should be higher than 40 %. Hence, when the plant material is fermented by solid-state fermentation in vessels, the control of the moisture content of the extruded substrate is of particular importance.

### V. Impact of an extrusion compared to a grinding

The following table 3 illustrates how the extrusion compared to a grinding. Indeed, the extrusion will affect, among others and as the grinding, the particle size of the substrate. The weight loss of the vessels content was measured during fermentation and at the harvesting time. In the table 3 below is reported the weight loss that occurred with the extruded substrate (compared to the control), compared to the weight loss of the unextruded substrate and the weight loss of the unextruded - grinded substrate.

**Table 3**

| | **Unextruded** | **Unextruded - Grinded** | **Extruded** |
|---|---|---|---|
| Ex2 Apple peels; moisture content 70% | 5.24% | No growth observed | 6.79% |
| Ex3 Sugar beet; moisture content 70% | 5.78% | No growth observed | 7.76% |
| Ex4 Orange; moisture content 70% | 6.08% | No growth observed | 6.87% |

Table 3 indicates that the grinding is not comparable to the extrusion when it comes to comparing the enhancement of the solid-state fermentation in vessels.

Whereas no growth was observed on the grinded substrate, the extruded substrate allows a clear enhancement of the fermentation compared to the unextruded substrate at 70% of moisture content.

### VI. Impact of co-substrate on fermentation

The following table 4 illustrates how the addition of a co-substrates, hence using at least two plant material from plants of different families, can improve the fermentation. The weight loss of the vessels content was measured during fermentation and at the harvesting time. In the table 4 below is reported the weight loss that occurred with the extruded substrate (compared to the control), depending on the absence of potatoes or the presence of 10% (90/10), 20% (80/20) or 30 % (70/30) in weight of potatoes in the extruded substrate.

**Table 4**

| | **Grapes/Potatoes 100/0** | **Grapes/Potatoes 90/10** | **Grapes/Potatoes 80/20** | **Grapes/Potatoes 70/30** |
|---|---|---|---|---|
| **Weight loss** | 3.03% | 3.9% | 4.87% | 4.89% |
| **Enhancement of weight loss** | na | + 28.7% | + 60.7% | + 61.4% |

Table 4 indicates that the addition of 10 % in weight of a second plant material as a co-substrate enhance the fermentation of 29% whereas an addition of at least 20% enhance the fermentation of 60% or more.

The method and the system developed in the invention makes it possible to produce a fermentation product at a large scale, i.e. in large quantities, without the drawbacks of the systems known in the state of the art. This result is obtained by an unknown and counter-intuitive approach in the field of bioprocessing. This approach can be presented as upscaling a solid-state fermentation by providing a high number of small bioreactors combined with an extruded substrate having a controlled moisture content instead of increasing the size of the bioreactors and fine-tuning the growth conditions.

The invention results in a highly efficient process, which has many advantages over the known processes, in terms of safety, environmental friendliness, ability to use by-products of the food industry and to accommodate seasonal variations of the available by-products, etc.

The invention is particularly adapted to the production of fermentation products for the agri-food industry.

From an economic standpoint, this use of small size vessels and extruded substrate to process a broader range of substrates allows modulation of operation to fit substrate specificities. In particular, this makes it possible to handle substrates of disparate volumes, heterogeneous sources, and for different purposes. For instance, sources of substrates may vary depending on seasonality and weather conditions. Flexibility of the process is of tremendous importance for an industrial plant to optimize capital expenditure use throughout the year.

The invention can be the subject of numerous variants and applications other than those described above. In particular, unless otherwise indicated, the different structural and functional characteristics of each of the implementations described above should not be considered as combined and / or closely and / or inextricably linked to each other, but on the contrary as simple juxtapositions. In addition, the structural and / or functional characteristics of the various embodiments described above may be the subject in whole or in part of any different juxtaposition or any different combination.

## Claims

1. Method (1) of solid-state fermentation of plant material to produce a fermentation product, said fermentation product comprising fermented plant material, the method comprising the steps of:
- providing (100) a substrate to be fermented (12), said substrate comprising plant material;
- extruding (300) the substrate to be fermented (12), said extruded substrate to be fermented having a moisture content higher than 40%;
- automatic vessels (42) filling (400) with the extruded substrate to be fermented, said vessels (42) have an inner volume of 100 L or less;
- automatic inoculation (500) of the extruded substrate with a microbial inoculant adapted to cause a solid-state fermentation of the extruded substrate, the inoculated vessels (52) being in a closed state after the inoculating step;
- storing (600) the inoculated vessels (52), being in a closed state, during a time sufficient for a fermentation of the extruded substrate;
- harvesting (700) the content of the inoculated vessels (52), said content comprising the fermented substrate and microbial biomass; and
- processing (800) the harvested content to obtain a fermentation product from all or part of the content of the inoculated vessels (52).

2. Method (1) according to claim 1, wherein the moisture content of the extruded substrate, before the automatic vessels filling (500), is comprised between 45% and 85%.

3. Method (1) according to claim 1 or 2, wherein the moisture content of the extruded substrate, before the automatic vessels filling (500), is at least 50%.

4. Method (1) according to anyone of claims 1 to 3, wherein the extrusion (300) of the substrate to be fermented (12) is conducted through opening having a cross section with an area value smaller than 2 cm².

5. Method (1) according to any one of claims 1 to 4, wherein the substrate to be fermented (12) comprises peels, skins, pulp, seeds, pomace, cake, and/or marc of fruit and/or vegetable.

6. Method (1) according to anyone of claims 1 to 5, wherein the substrate to be fermented (12) comprises plant material from plants of different families.

7. Method (1) according to anyone of claims 1 to 6, wherein the filling step is done at a rate of at least 50 vessels per hour.

8. Method (1) according to anyone of claims 1 to 7, further comprising a step of killing microorganisms (450) before the automatic inoculation step (500) occurs, said step of killing microorganisms (450) being applied to empty vessels (42), filed vessels, plant material and/or the substrate to be fermented (12) before the filing of the vessels (42).

9. Method (1) according to anyone of claims 1 to 8, wherein the fermentation product comprises: spores, enzymes, pigments, flavours, fragances, microbial biomass, and/or antimicrobials.

10. Method (1) according to anyone of claims 1 to 9, further comprising a step of closing (440) the vessels with a barrier system after the step of filling (400) and/or after the step of inoculation (500).

11. Method (1) according to anyone of claims 1 to 10, wherein said microbial inoculant adapted to cause a solid-state fermentation of the substrate comprises at least one microorganism belonging to the taxonomic class *Bacilli.*

12. Method (1) according to anyone of claims 1 to 11, wherein said microbial inoculant adapted to cause a solid-state fermentation of the substrate comprises at least one microorganism belonging to the genus *Brettanomyces* or *Aureobasidium.*

13. Method (1) according to anyone of claims 1 to 12, wherein said microbial inoculant adapted to cause a solid-state fermentation of the substrate comprises at least one Dikarya fungus.

14. Method (1) according to anyone of claims 1 to 13, wherein the inoculated vessels (52) in the closed state are stored in a non-aseptic environment.

15. A system (2) for solid-state fermentation of plant material to produce a fermentation product, said fermentation product comprising fermented plant material, said system comprising:
- a supply device (10) arranged to receive a substrate to be fermented (12), said substrate to be fermented comprising plant material;
- an extruder (30) arranged to extrude the substrate to be fermented (12) at a moisture content higher than 40%;
- an automated filling device (40) arranged to fill vessels (42) with the extruded substrate, said vessels (42) having an inner volume of 100 L or less;
- an automatic inoculating device (50) of the extruded substrate with a microbial inoculant adapted to cause a solid-state fermentation of the extruded substrate;
- an incubation chamber (60) arranged to allow a solid-state fermentation of the extruded substrate by the microbial inoculant in the inoculated vessels (52), the vessels being in a closed state;
- an automated harvesting device (70) arranged to harvest the content of the inoculated vessels (52), said content comprising the fermented substrate and microbial biomass; and
- automated processing device (80) configured to process the harvested content to obtain a fermentation product from all or part of the content of the inoculated vessels (52).
